(19) Europäisches Patentamt
European Patent Office
Office européen des brevets

(11) EP 4 660 183 A1

(12) **EUROPEAN PATENT APPLICATION**
published in accordance with Art. 153(4) EPC

(43) Date of publication:
**10.12.2025 Bulletin 2025/50**

(21) Application number: **24749439.6**

(22) Date of filing: **26.01.2024**

(51) International Patent Classification (IPC):
***C07C 233/64*** (2006.01) ***C07C 243/38*** (2006.01)
***C07C 243/16*** (2006.01) ***C07C 15/00*** (2006.01)
***C07C 15/12*** (2006.01) ***C07D 213/00*** (2006.01)
***C07D 401/00*** (2006.01) ***C07D 403/00*** (2006.01)
***A61K 31/166*** (2006.01) ***A61P 25/02*** (2006.01)
***A61P 25/04*** (2006.01) ***A61P 29/00*** (2006.01)

(52) Cooperative Patent Classification (CPC):
**A61K 31/166; A61P 25/02; A61P 25/04; A61P 29/00; C07C 15/00; C07C 15/12; C07C 233/64; C07C 243/16; C07C 243/38; C07D 213/00; C07D 401/00; C07D 403/00**

(86) International application number:
**PCT/BR2024/050026**

(87) International publication number:
**WO 2024/159286 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.01.2023 US 202363482211 P**

(71) Applicants:
- **Eurofarma Laboratórios S.A.**
  **06696-000 São Paulo - SP (BR)**
- **Universidade Federal Do Rio De Janeiro - UFRJ**
  **21941-853 Rio de Janeiro -RJ (BR)**

(72) Inventors:
- **BARREIRO, Gabriela**
  **SP 04514-032 São Paulo (BR)**
- **SANT'ANA, Danilo Pereira De**
  **SP 06709-320 São Paulo (BR)**
- **MONTEIRO, Júlia Lammoglia**
  **SP 06704175 São Paulo (BR)**
- **GAMBA, Luis Eduardo Reina**
  **SP 06404-326 São Paulo (BR)**
- **FRAGA, Carlos Alberto Manssour**
  **(deceased) (BR)**
- **BARREIRO, Eliezer Jesus De Lacerda**
  **(deceased) (BR)**
- **LIMA, Lídia Moreira**
  **RJ 21931-220 Rio de Janeiro (BR)**

(74) Representative: **Engelhard, Markus**
**Boehmert & Boehmert**
**Anwaltspartnerschaft mbB**
**Pettenkoferstrasse 22**
**80336 München (DE)**

(54) **NAV1.7- AND/OR NAV1.8-INHIBITING PHENOLIC COMPOUNDS, PROCESSES FOR THE PREPARATION THEREOF, COMPOSITIONS, USES, METHODS FOR TREATMENT USING SAME, AND KITS**

(57) The present invention relates to phenolic compounds blocking Nav 1.7 and/or Nav 1.8. More specifically, the present invention is related to phenols comprising Formula (I), in which the substituents $R_1$ to $R_{12}$ are selected independently of the groups defined in the specification, as well as their processes of preparation, compositions comprising at least one of these compounds, uses, treatment methods for treating or preventing pain-related pathologies and kits. The present invention belongs to the fields of medicinal chemistry, organic synthesis, as well as to the treatment of pain-related diseases.

EP 4 660 183 A1

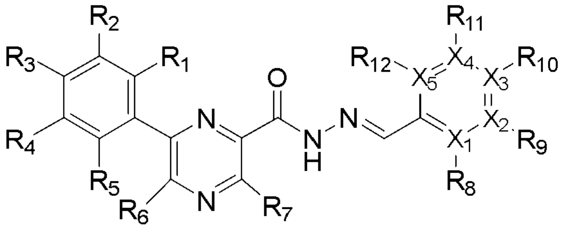

Formula (I)

## Description

## FIELD OF INVENTION

The present invention refers to phenolic compounds blocking Nav 1.7 and/or Nav 1.8, processes for the preparation thereof, compositions containing these, uses, kits and treatment methods to treat or prevent pain-related pathologies. The present invention belongs to the fields of medicinal chemistry, organic synthesis, as well as to the treatment of pain-related diseases.

## BACKGROUND OF INVENTION

Physiological pain is an important protective mechanism designed to alert the body to real or potential injuries that can put the integrity thereof at risk. Broadly speaking, physiological pain can be classified as nociceptive pain and inflammatory pain. Nociceptive pain is characterized by having a high activation threshold, which remains until the stimulus that generated it is eliminated. Inflammatory pain, which arises as a response to tissue damage, is characterized by having a low activation threshold and is a consequence of the activity of molecular mediators of the inflammatory process in sensitizing nociceptors (Schaible. Langenbecks Arch. Surg. 2004, 389, 237). When these nociceptive processes remain in the absence of noxious stimuli or in response to non noxious stimuli, the protective and repair role of pain loses its functionality, configuring a maladaptive picture of neural plasticity and, as a consequence, a pathological state of chronic pain. Among the syndromes included in this classification, neuropathic pain has a high prevalence and impact today (Smith. Pain. 2020, 161, 1:S127; Cavalli. Int. J. Immunopathol. Pharmacol. 2019, 33:2058738419838383; Bouhassira. Rev Neurol (Paris). 2019, 175(1-2):16; Scholz. Nature Neurosci., 2002, 5,1062; Costigan. Annu. Rev. Neurosci., 2009, 32, 1).

Neuropathic pain is defined by the International Association for the Study of Pain (IASP) as pain initiated or caused by a primary dysfunction or injury in the central and/or peripheral nervous system (Dworkin. Clin. J. Pain, 2002, 18(6), 343). Central neuropathic pain comes from spinal cord injuries or central nervous system diseases such as multiple sclerosis or Parkinson's disease (Ducreux. Brain, 2006, 129, 963). Peripheral neuropathic pain, on the other hand, can be caused by trauma, metabolic disorders, chemical neurotoxicity, infection, or tumor invasion, among others. Among the most common syndromes of neuropathic pain are chemotherapy-induced neuropathic pain, complex regional pain, neuropathy related to viral infection, neuropathy secondary to tumor infiltration, diabetic neuropathy, phantom limb pain, postherpetic neuralgia, trigeminal neuralgia, and postsurgical neuralgia (Pak. Curr. Pain Headache Rep., 2018, 22(2), 9).

Currently, there is no specific treatment for the control of pathologies related to neuropathic pain, however, the first-line alternative consists of the use of opioid analgesics, and - as adjuvants - local anesthetics, anticonvulsants and antidepressants. However, the adverse effects and low efficacy drastically limit the use of these agents in the control of various pain-related pathologies (Kushnarev. Expert Opin. Investig. Drugs, 2020, 29(3), 259; Emery. Expert Opin. Ther. Targets, 2016, 20(8), 975).

Voltage-gated sodium channels (Nav) play a key role in the transmission of pain-related stimuli. These channels are activated in response to membrane depolarization, allowing the generation and propagation of action potentials in neurons (and other electrically excitable cells), by controlling the flow of sodium ions through the membranes. Structurally, voltage-gated sodium channels are heteromeric transmembrane proteins consisting of one $\alpha$ subunit and two $\beta$ auxiliary subunits. The $\alpha$ subunit is organized into four homologous domains (I-IV), each with six transmembrane segments (S1-S6). The S4 segment of each domain is characterized by presenting a conserved region of arginine residues, which act as sensors of the intra- and extracellular electrical environment of the neuron. This mechanism makes it possible to transform changes in the cellular electric field into specific conformational changes that, in turn, regulate the activation, deactivation and inactivation of voltage-gated sodium channels (Catterall. Nat. Chem. Biol., 2020, 16, 1314; Wisedchaisri. Cell., 2019, 178(4), 993; Clairfeuille. Science, 2019, 363, 1302).

In mammals, nine subunits $\alpha$ (Nav 1.1 - Nav 1.9) and four auxiliary subunits $\beta$ ($\beta$1-$\beta$4) have been identified. The $\alpha$ subunits can also be classified according to their susceptibility to blocking by tetrodotoxin (TTX), being classified as sensitive to tetrodotoxin (Nav 1.1, Nav 1.2, Nav 1.3, Nav 1.4, Nav 1.6 and Nav 1.7) or resistant to tetrodotoxin (Nav 1.5, Nav 1.8 and Nav 1.9) (Lera-Ruiz. J. Med. Chem., 2015, 58(18), 7093; Bagal. J. Med. Chem., 2013, 56(3), 593). Each of these $\alpha$ subunits has a different profile of expression and function, so that some of them are essential for the proper functioning of organs such as the heart and/or brain. Thus, the non-selective blocking of these channels is related to several types of adverse effects, such as migraine, epilepsy, paralysis and muscle and cardiac syndromes, among others (Bagal. J. Med. Chem., 2013, 56(3), 593; Bagal. Channels, 2015, 9(6), 360).

Broadly speaking, sodium channels are distributed mainly in the central and peripheral nervous system, in neurons and glia. Nav channels 1.1, 1.2, and 1.3 are primarily expressed in the brain. The Nav 1.4 and Nav 1.5 channels are found primarily in skeletal and cardiac muscles, respectively. Nav 1.6 channels are expressed in the central and peripheral nervous systems, while Nav 1.9 channels are selectively expressed in C-type nociceptive fibers in the dorsal

root ganglion. On the other hand, the Nav 1.7 and Nav 1.8 channels are mainly found in the peripheral nervous system and are directly related to the processes of pain transmission (Law. Drug Discovery Today, 2019, 24(7), 1389; Bagal. Channels (Austin), 2015, 9(6), 360; Lera-Ruiz. J. Med. Chem., 2015, 58 (18), 7093).

Nav 1.7 sodium channels are expressed broadly in the olfactory epithelium, sympathetic ganglion, and dorsal root ganglion, predominantly in nociceptive fibers C and A$\delta$. A large amount of evidence supports the important role of Nav 1.7 sodium channels in pain transmission processes. For example, gain-of-function related mutations in the gene (SCN9A), which encodes sodium channel Nav 1.7, are associated with extreme pain disorders such as congenital pain insensitivity, paroxysmal extreme pain disorder, and primary erythromelalgia. On the other hand, mutations related to loss of gene function (SCN9A) are related to congenital insensitivity to pain in individuals who, in general terms, are free of motor or cognitive impairment (Vetter. Pharmacology & Therapeutics, 2017, 172, 73; Ahuja. Science, 2015, 350(6267), 1491; Kingwell. Nat. Rev. Drug Discov., 2019, 18, 321; Safina. J. Med. Chem., 2021, 64, 2953; Luo. J. Med. Chem., 2019, 62, 831; Bankar. Cell Reports, 2018, 24, 3133).

Nav 1.8 sodium channels are most expressed in the peripheral nervous system, widely (but not exclusively) in C-type nociceptive fibers in the dorsal root ganglion. Recent evidence including elevated expression levels of Nav 1.8 in chronic pain states, data with Nav 1.8 *knockout* animals, and analgesic activity of Nav 1.8-specific desensitizing oligodeoxynucleotides, among others (Brown. Bioorg. Med. Chem., 2019, 27(1), 230; Payne. Br. J. Pharmacol., 2015, 172(10), 2654; Bagal. Med. Chem. Lett. 2015, 6(6) 650; Kort. J. Med. Chem. 2008, 51, 407; Zhang. Neuropharmacology, 2010, 59, 201 and 207), support the role of the sodium channel Nav 1.8 in the development and process of pain-related pathologies (Kingwell. Nat. Rev. Drug Discov., 2019, 18, 321; Law. Drug Discovery Today, 2019, 24(7), 1389; Bagal. Channels (Austin), 2015, 9(6), 360; Lera-Ruiz. J. Med. Chem., 2015, 58(18), 7093).

Thus, the voltage-gated sodium channels Nav 1.7 and Nav 1.8 are considered promising therapeutic targets for the treatment of neuropathic pain-related dysfunctions (Kornecook. J. Pharmacol. Exp. Ther., 2017, 362, 146; Kingwell. Nat. Rev. Drug Discov., 2019, 18, 321; Bagal. Channels (Austin), 2015, 9(6), 360; Lera-Ruiz. J. Med. Chem., 2015, 58(18), 7093; Deuis. Neuropharmacology, 2017, 127, 87 and 108; Kushnarev. Expert Opin. Investig. Drugs, 2020, 29(3), 259; McKerrall. Bioorganic & Medicinal Chemistry Lett., 2018, 28, 3141; Emery. Expert Opin. Ther. Targets, 2016, 20(8), 975; Bagal. Bioorganic & Medicinal Chemistry Lett., 2014, 24, 3690).

A large number of compounds have been described in the literature by their ability to act as blockers of Nav 1.7 and 1.8 sodium channels, however, they present a great structural diversity, which fact that does not allow the establishment of a common pharmacophoric group.

The patent literature contains several examples of compounds that act as sodium channel blockers. In particular, Nav 1.7 selective sodium channel blockers are described in US10550080, US9765029, and US10000475. Additionally, some documents describe selective blockers of Nav 1.8 sodium channels such as WO2020261114, WO2020092667, US9163042, WO2014120808, WO2014120815, WO2018213426, WO2019014352, WO2015006280, and US7928107. These documents reveal compounds with different structures from the present invention.

In addition, there are patent documents that describe dual Nav 1.7 and 1.8 blockers, including WO2018235851, US8629149, JP2017001991 that reveal, respectively, pyridyl amines, oxopiperazine derivatives and benzoxazolons. However, all these documents reveal compounds with structures and physicochemical characteristics different from the present invention.

In this context, it is advantageous to develop new alternatives of compounds that can act as Nav 1.7 and/or Nav 1.8 blockers that have adequate pharmacological action and, preferably, provide mitigated adverse effects. Therefore, the present invention refers to phenols as an alternative and/or complement to the treatment of pain-related diseases.

## SUMMARY OF THE INVENTION

The present invention discloses phenols with blocking activity of Nav 1.7 and/or 1.8 channels against pain-related pathologies, as well as related compositions, uses, kits, treatment methods and preparation processes.

The present invention refers to compound(s) of Formula (I):

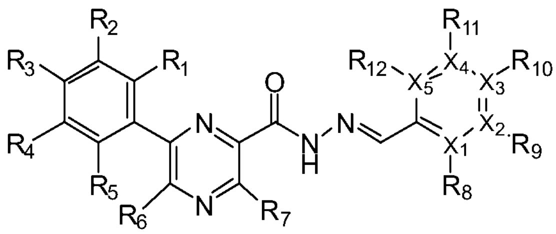

Formula (I)

or a pharmaceutically acceptable salt, hydrate, solvate, ester and isomer thereof, wherein:

- one of $X_1$, $X_2$, $X_3$, $X_4$ and $X_5$ is nitrogen and the others are carbon, where when one of $X_1$, $X_2$, $X_3$, $X_4$ and $X_5$ is nitrogen, the corresponding R ($R_5$, $R_6$ and/or $R_7$) will be null;
- $R_1$, $R_2$, $R_3$, $R_4$, and $R_5$ are independently selected from the group consisting of hydrogen, halogen, hydroxy, alkoxy $C_1$-$C_5$ linear or branched, alkyl $C_1$-$C_6$ linear or branched, wherein at least one of $R_1$-$R_5$ is hydroxy, when $R_6$-$R_{12}$ are different from hydroxy;
- $R_6$ and $R_7$ are independently selected from the group consisting of hydrogen, halogen, hydroxy, $C_1$-$C_6$ linear or branched alkoxy, linear or branched $C_1$-$C_6$ alkyl, wherein at least one of $R_6$-$R_7$ is hydroxy, when $R_1$-$R_5$ and $R_8$-$R_{12}$ are different from hydroxy; and
- $R_8$, $R_9$, $R_{10}$, $R_{11}$, and $R_{12}$ are independently selected from the group consisting of hydrogen, linear or branched $C_1$-$C_5$ alkoxy, alkyl linear or branched $C_1$-$C_6$; halogen, hydroxy, wherein at least one of $R_8$-$R_{12}$ is hydroxy, while $R_1$-$R_7$ are different from hydroxy.

The present invention refers to compositions comprising one or more compound(s) of Formula (I) or a salt, hydrate, solvate and pharmaceutically acceptable isomer thereof; and one or more pharmaceutically acceptable excipients.

In addition, the kits, according to the present invention, may comprise such compositions and application devices, which may include ampoules, syringes and others. Alternatively, the kits according to this invention comprise more than one compound of Formula (I) arranged in one or more dosage forms, including without limitation, tablets, accompanied by administration instructions.

The present invention further refers to methods of treatment, prevention, relief, suppression and/or control of diseases related to neuropathic pain. Uses of Formula (I) compound(s) to prepare a drug for the treatment of pathologies related to neuropathic pain are also taught. Finally, the present invention teaches processes for obtaining compound(s) of Formula (I).

In addition, some compounds described in this invention may exist as tautomers, so the individual tautomers, as well as their mixtures, are included in the compounds with structural formula I.

## DETAILED DESCRIPTION OF THE INVENTION

The present invention presents, in a first embodiment, compounds of Formula (I):

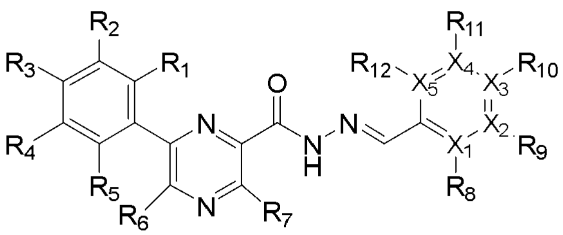

Formula (I)

or a pharmaceutically acceptable salt, hydrate, solvate and isomer thereof, wherein:

- one of $X_1$, $X_2$, $X_3$, $X_4$ and $X_5$ is nitrogen and the others are carbon, where when one of $X_1$, $X_2$, $X_3$, $X_4$ and $X_5$ is nitrogen, the corresponding R ($R_5$, $R_6$ and/or $R_7$) will be null;
- $R_1$, $R_2$, $R_3$, $R_4$, and $R_5$ are independently selected from the group consisting of hydrogen, halogen, hydroxy, alkoxy $C_1$-$C_5$ linear or branched, alkyl $C_1$-$C_6$ linear or branched, wherein at least one of $R_1$-$R_5$ is hydroxy, when $R_6$-$R_{12}$ are different from hydroxy;
- $R_6$ and $R_7$ are independently selected from the group consisting of hydrogen, halogen, hydroxy, $C_1$-$C_6$ linear or branched alkoxy, linear or branched $C_1$-$C_6$ alkyl, wherein at least one of $R_6$-$R_7$ is hydroxy, when $R_1$-$R_5$ and $R_8$-$R_{12}$ are different from hydroxy; and
- $R_8$, $R_9$, $R_{10}$, $R_{11}$, and $R_{12}$ are independently selected from the group consisting of hydrogen, linear or branched $C_1$-$C_5$ alkoxy, alkyl linear or branched $C_1$-$C_6$, halogen, hydroxy, where at least one of $R_8$-$R_{12}$ is hydroxy, while $R_1$-$R_7$ are different from hydroxy.

As described herein, the compounds of the present invention comprise multiple variable groups (R, X, etc.). As a person skilled in the matter will recognize, the group combinations contemplated by this invention are those combinations that result in the formation of stable or chemically viable compounds.

The term "stable" in this context refers to compounds that are not substantially altered when subjected to conditions that allow their production, detection and preferably their recovery, purification and use for one or more of the

purposes disclosed herein.

In some embodiments, a stable compound or chemically viable compound is one that is not substantially altered when maintained at a temperature of 40 °C or less, in the absence of moisture or other chemically reactive conditions, for at least one week.

In an embodiment, one of $X_1$, $X_2$, $X_3$, $X_4$ and $X_5$ is nitrogen and the others are carbon, where when one of $X_1$, $X_2$, $X_3$, $X_4$ and $X_5$ is nitrogen, the corresponding R ($R_5$, $R_6$, and/or $R_7$) is null; $R_1$ is selected from the group consisting of hydrogen, halogen, and hydroxy; $R_2$ is hydrogen; $R_3$ is selected from the hydroxy and alkoxy group $C_1$-$C_5$ linear or branched; $R_4$ is selected from the hydrogen and hydroxy group; $R_5$ is hydrogen; $R_6$ is selected from the hydrogen and hydroxy group; $R_7$ is selected from the hydrogen and hydroxy group; $R_8$ is selected from the hydrogen and hydroxy group, or is null when $X_1$ is nitrogen; $R_9$ is selected from the group consisting of hydrogen, hydroxy, linear or branched $C_1$-$C_5$ alkoxy, and linear or branched $C_1$-$C_6$alkyl; $R_{10}$ is selected from the group consisting of hydrogen and hydroxy or is null when $X_3$ is nitrogen; $R_{11}$ is selected from the group consisting of hydrogen, hydroxy, linear or branched $C_1$-$C_6$ alkyl, and $C_1$-$C_6$ linear or branched alkoxy, or it is null when $X_4$ is nitrogen; $R_{12}$ is selected from the group consisting of hydrogen, hydroxy, and halogen, or it is null when $X_5$ is nitrogen.

In one embodiment, $R_1$ is selected from the group consisting of hydrogen, chlorine, bromine, fluorine, and hydroxy. In a preferred embodiment, $R_1$ is selected from the group consisting of hydrogen, fluorine, and hydroxy.

In one embodiment, $R_2$ is hydrogen.

In an embodiment, $R_3$ is selected from the group consisting of hydroxy, methoxy, ethoxy; propoxy, isopropoxy, n-butoxy, sec-butoxy, isobutoxy, tert-butoxy, n-pentoxy, sec-pentoxy, tert-pentoxy, but not limited to same. In a preferred embodiment, $R_3$ is selected from the group consisting of hydroxy, methoxy, and ethoxy.

In one embodiment, $R_4$ is selected from the group consisting of hydrogen and hydroxy.

In one embodiment, $R_5$ is hydrogen.

In one embodiment, $R_6$ is selected from the group consisting of hydrogen and hydroxy.

In one embodiment, $R_7$ is selected from the group consisting of hydrogen and hydroxy.

In an embodiment, $R_8$ is selected from the group consisting of hydrogen and hydroxy or is null when $X_1$ is nitrogen.

In an embodiment, $R_9$ is selected from the group consisting of hydrogen, hydroxy, methyl, ethyl, propyl, isopropyl, n-butyl, sec-butyl, tert-butyl, n-pentyl, sec-pentyl, tert-pentyl, methoxy, ethoxy; propoxy, isopropoxy, n-butoxy, sec-butoxy, isobutoxy, tert-butoxy, n-pentoxy, sec-pentoxy, tert-pentoxy, but not limited to same. In a preferred embodiment, $R_9$ is selected from the group consisting of hydrogen, hydroxy, methyl, and methoxy.

In an embodiment, $R_{10}$ is selected from the group consisting of hydrogen and hydroxy or is null when $X_3$ is nitrogen.

In an embodiment, $R_{11}$ is selected from the group consisting of hydrogen, hydroxy, methyl, ethyl, propyl, isopropyl, n-butyl, sec-butyl, tert-butyl, n-pentyl, sec-pentyl, tert-pentyl, methoxy, ethoxy; propoxy, isopropoxy, n-butoxy, sec-butoxy, iso-butoxy, tert-butoxy, n-pentoxy, sec-pentoxy, tert-pentoxy but not being limited to same, or $R_{11}$ is null when $X_4$ is nitrogen. In a preferred embodiment, $R_{11}$ is selected from the group consisting of hydrogen, hydroxy, methyl, methoxy, or null when $X_4$ is nitrogen.

In one embodiment, $R_{12}$ is selected from the group consisting of hydrogen, hydroxy, fluorine, chlorine, bromine, or is null when $X_5$ is nitrogen. In a preferred embodiment, $R_{12}$ is selected from the group consisting of hydrogen, hydroxy, fluorine, or is null when $X_5$ is nitrogen.

In an implementation of the first embodiment, the Formula (I) compound(s) is selected from the group(s) consisting of:

(*E*)-*N'*-(3,5-dimethoxybenzylidene)-6-(4-hydroxyphenyl)pyrazine-2-carbohydrazide (Compound 1);
(*E*)-*N'*-(3,5-dimethylbenzylidene)-6-(4-hydroxyphenyl)pyrazine-2-carbohydrazide (Compound 2)
(*E*)-*N'*-(2-fluoro-5-methoxybenzylidene)-6-(4-hydroxyphenyl)pyrazine-2-carbohydrazide (Compound 3);
(*E*)-6-(4-hydroxyphenyl)-*N'*-((5-hydroxypyridine-3-yl)methylene)pyrazine-2-carbohydrazide (Compound 4);
(*E*)-*N'*-(3,5-dimethoxybenzylidene)-6-(2-hydroxy-4-methoxyphenyl)pyrazine-2-carbohydrazide (Compound 5);
(*E*)-*N'*-(3,5-dimethoxybenzylidene)-6-(3-hydroxy-4-methoxyphenyl)pyrazine-2-carbohydrazide (Compound 6);
(*E*)-*N'*-(3,5-dimethylbenzylidene)-6-(2-hydroxy-4-methoxyphenyl)pyrazine-2-carbohydrazide (Compound 7);
(*E*)-6-(4-ethoxy-2-hydroxyphenyl)-*N'*-(2-fluoro-5-methoxybenzylidene)pyrazine-2-carbohydrazide (Compound 8);
(*E*)-6-(4-ethoxy-2-hydroxyphenyl)-*N'*-((2-fluoro-5-methoxypyridine-3-yl)methylene)pyrazine-2-carbohydrazide (Compound 9);
(*E*)-6-(4-ethoxy-3-hydroxyphenyl)-*N'*-((2-fluoro-5-methoxypyridine-3-yl)methylene)pyrazine-2-carbohydrazide (Compound 10);
(*E*)-6-(4-ethoxy-3-hydroxyphenyl)-*N'*-(2-fluoro-5-methoxybenzylidene)pyrazine-2-carbohydrazide (Compound 11);
(*E*)-*N'*-(3,5-dimethoxybenzylidene)-5-hydroxy-6-(4-methoxyphenyl)pyrazine-2-carbohydrazide (Compound 12);
(*E*)-*N'*-(3,5-dimethylbenzylidene)-5-hydroxy-6-(4-methoxyphenyl)pyrazine-2-carbohydrazide (Compound 13);
(*E*)-*N'*-(3,5-dimethoxybenzylidene)-3-hydroxy-6-(4-methoxyphenyl)pyrazine-2-carbohydrazide (Compound 14);

(*E*)-*N'*-(3,5-dimethylbenzylidene)-3-hydroxy-6-(4-methoxyphenyl)pyrazine-2-carbohydrazide (Compound 15);
(*E*)-6-(4-ethoxyphenyl)-*N'*-((2-fluoro-5-methoxypyridine-3-yl)methylene)-5-hydroxypyrazine-2-carbohydrazide (Compound 16);
(*E*)-6-(4-ethoxyphenyl)-*N'*-((2-fluoro-5-methoxypyridine-3-yl)methylene)-3-hydroxypyrazine-2-carbohydrazide (Compound 17);
(*E*)-6-(4-ethoxy-2-fluorophenyl)-5-hydroxy-N'-(3-methoxybenzylidene)pyrazine-2-carbohydrazide (Compound 18);
(*E*)-6-(4-ethoxy-2-fluorophenyl)-3-hydroxy-*N'*-(3-methoxybenzylidene)pyrazine-2-carbohydrazide (Compound 19);
(*E*)-*N'*-(3,5-dihydroxybenzylidene)-6-(4-methoxyphenyl)pyrazine-2-carbohydrazide (Compound 20);
(*E*)-*N'*-(3-hydroxy-5-methoxybenzylidene)-6-(4-methoxyphenyl)pyrazine-2-carbohydrazide (Compound 21);
(*E*)-*N'*-(4-hydroxybenzylidene)-6-(4-methoxyphenyl)pyrazine-2-carbohydrazide (Compound 22);
(*E*)-*N'*-(3-hydroxybenzylidene)-6-(4-methoxyphenyl)pyrazine-2-carbohydrazide (Compound 23);
(*E*)-*N'*-(2-hydroxybenzylidene)-6-(4-methoxyphenyl)pyrazine-2-carbohydrazide (Compound 24);
(*E*)-*N'*-(3-hydroxy-5-methylbenzylidene)-6-(4-methoxyphenyl)pyrazine-2-carbohydrazide (Compound 25);
(*E*)-6-(4-ethoxyphenyl)-*N'*-(3-hydroxy-5-methylbenzylidene)pyrazine-2-carbohydrazide (Compound 26);
(*E*)-6-(4-ethoxyphenyl)-*N'*-(2-fluoro-5-hydroxybenzylidene)pyrazine-2-carbohydrazide (Compound 27);
(*E*)-6-(4-ethoxyphenyl)-*N'*-(2-hydroxy-5-methoxybenzylidene)pyrazine-2-carbohydrazide (Compound 28);
(*E*)-6-(4-ethoxyphenyl)-*N'*-((5-hydroxypyridine-3-yl)methylene)pyrazine-2-carbohydrazide (Compound 29);
(*E*)-6-(4-ethoxyphenyl)-*N'*-((6-hydroxypyridine-2-yl)methylene)pyrazine-2-carbohydrazide (Compound 30);
(*E*)-6-(4-ethoxyphenyl)-*N'*-(2-hydroxy-3,5-dimethoxybenzylidene)pyrazine-2-carbohydrazide (Compound 31);
(*E*)-6-(4-ethoxyphenyl)-*N'*-((2-fluoro-5-hydroxypyridine-3-yl)methylene)pyrazine-2-carbohydrazide (Compound 32);
(*E*)-6-(4-ethoxyphenyl)-*N*'-((2-fluoro-4-hydroxy-5-methoxypyridine-3-yl)methylene)pyrazine-2-carbohydrazide (Compound 33);
(*E*)-6-(4-ethoxyphenyl)-*N*'-((6-hydroxy-4-methoxypyridine-2-yl)methylene)pyrazine-2-carbohydrazide (Compound 34);
(*E*)-6-(4-ethoxyphenyl)-*N*'-((2-hydroxy-5-methoxypyridine3-yl)methylene)pyrazine-2-carbohydrazide (Compound 35); and
(*E*)-6-(4-ethoxyphenyl)-*N*'-((2-hydroxy-6-methoxypyridine-4-yl)methylene)pyrazine-2-carbohydrazide (Compound 36).

In an implementation of the first embodiment, the Formula (I) compound(s) are voltage-gated sodium channel blockers Nav 1.7 and/or Nav 1.8. In a preferred embodiment, the Formula (I) compound(s) are dual voltage-gated sodium channel blockers Nav 1.7 and Nav 1.8.

In an implementation of the first embodiment, the compound(s) of Formula (I) may have a basic nature and, consequently, pharmaceutically acceptable salts may be obtained by the addition of organic or inorganic acids. Nonlimiting examples of organic acids that can be used are fumaric, maleic, benzoic, lactic acids, among others. Among the inorganic acids, we can mention hydrochloric acid, phosphoric acid, hydrobromic acid, sulfuric acid, nitric acid, among others.

In an implementation of the first embodiment, the compound(s) of Formula (I) can be obtained in the form of crystals, which can be optionally presented as pharmaceutically acceptable solvates, in which the solvent is incorporated in stoichiometric proportions or not into the crystal lattice. In further embodiments, the crystallization solvent is water, resulting in pharmaceutically acceptable hydrates.

Finally, in an implementation of the first embodiment, the Formula (I) compound(s) may present more than one isomer, including without limitation, spatial isomerism, such as geometric and optical isomerism.

**DEFINITIONS:**

In a first embodiment, the present invention presents phenolic compounds blocking Nav 1.7 and/or Nav 1.8 of Formula (I), or a salt, hydrate, solvate and pharmaceutically acceptable isomer thereof.

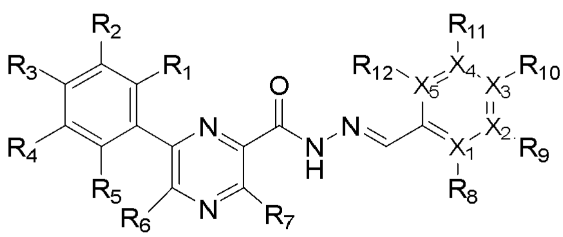

Formula (I)

In order to clarify or elucidate the terms used in this invention, the following definitions are presented, whereby the scope is not limited thereto.

The term "halogen" refers to the elements of the 7A family of the periodic table, which are: fluorine (F), chlorine (Cl), bromine (Br), iodine (I), astatine (At) and tennessine (Ts).

The term "linear or branched $C_1$-$C_6$ alkyl refers to saturated straight- or branched-chain hydrocarbons such as for example, methyl, ethyl, propyl, isopropyl, n-butyl, sec-butyl, tert-butyl, n-pentyl, n-pentyl, sec-pentyl, tert-pentyl, but not limited to them.

The term "linear or branched $C_1$-$C_6$ alkoxy" refers to alkyl groups attached to an oxygen radical, such as methoxy, ethoxy; propoxy, isopropoxy, n-butoxy, sec-butoxy, iso-butoxy, tert-butoxy, n-pentoxy, sec-pentoxy, tert-pentoxy, but not limited to same.

In a second embodiment, the present invention presents a composition comprising a therapeutically effective amount of compound(s) of Formula (I) of the present invention or a pharmaceutically acceptable salt, hydrate, solvate, and isomer thereof; and one or more pharmaceutically acceptable excipients.

Pharmaceutically acceptable excipients are any substance, other than the active pharmaceutical ingredient, that has been evaluated for its safety and that is intentionally added to the dosage form. Such excipients are selected according to the pharmaceutical dosage form of interest, their route of administration, physicochemical compatibility with the active ingredient, and the effect on efficacy.

In addition, these excipients are widely known in the state of the art and are classified according to their function, including without limitation diluents, binders, disintegrants or disaggregators, lubricants, suspending agents, thickeners, solvents, surfactants, sliders, anti-caking agents or flow agents, glazing agents, plasticizers, sweeteners, isotonicity agents, dyes and pigments, preservatives, antioxidants, modifying agents or pH control, complexing agents, chelating agents, flavorings, viscosity modifying agents, opacifiers, permeation promoters, among others.

In an implementation of the second embodiment, the pharmaceutical compositions of the present invention can be administered by several routes including oral, sublingual, nasal, parenteral, injectable, submuscular, topical, transdermal, ocular, rectal, but not limited to these.

In a third embodiment, the present invention presents the use of compound(s) of Formula (I) or a salt, hydrate, solvate and pharmaceutically acceptable isomer thereof to prepare a drug to treat pathologies related to neuropathic pain.

In an implementation of the third embodiment, said pathologies are selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia.

In a fourth embodiment, the present invention presents a method of treatment, prevention, relief, suppression and/or control of pathologies related to neuropathic pain comprising the administration of an effective amount of Formula (I) compound(s) or a salt, hydrate, solvate and pharmaceutically acceptable isomer thereof.

In an implementation of the fourth embodiment, the method is for the treatment, prevention, relief, suppression and/or control of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia.

In another implementation of the fourth embodiment, the administration of at least one Formula (I) compound is selected from the group comprising oral, sublingual, nasal, parenteral, injectable, submuscular, topical, transdermal, ocular and rectal routes.

In a fifth embodiment, the present invention presents processes for obtaining compound(s) of Formula (I) comprising the following steps:

(a) Formation of the Formula III intermediate:

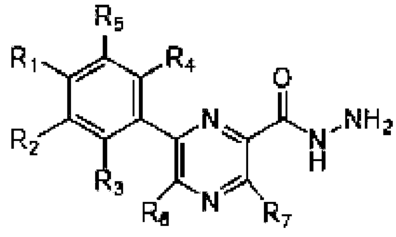

Formula III

from the hydrazinolysis reaction of a Formula IV intermediate:

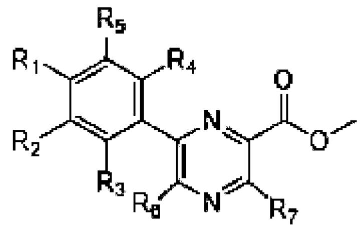

Formula IV

(b) obtaining Formula I compound;

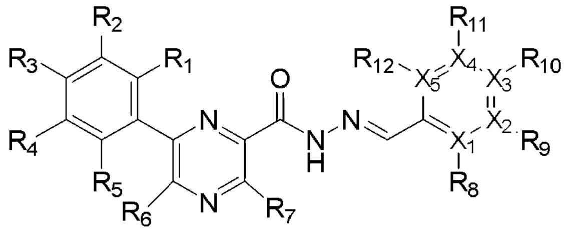

Formula (I)

from the condensation of Formula II intermediates:

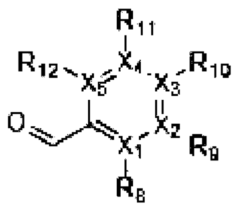

Formula II

and Formula III with or without the presence of a catalyst and a suitable solvent;
wherein,

- one of $X_1$, $X_2$, $X_3$, $X_4$ and $X_5$ is nitrogen and the others are carbon, wherein when one of $X_1$, $X_2$, $X_3$, $X_4$ and $X_5$ is nitrogen, the corresponding R ($R_5$, $R_6$ and/or $R_7$) will be null;
- $R_1$, $R_2$, $R_3$, $R_4$, and $R_5$ are independently selected from the group consisting of hydrogen, halogen, hydroxy, alkoxy $C_1$-$C_6$ linear or branched, alkyl $C_1$-$C_6$ linear or branched alkoxy, wherein at least one of $R_1$-$R_5$ is hydroxy, when $R_6$-$R_{12}$ are different from hydroxy;
- $R_6$ and $R_7$ are independently selected from hydrogen, halogen, hydroxy, $C_1$-$C_5$ linear or branched alkyl, $C_1$-$C_6$ linear or branched alkoxy, wherein at least one of $R_{6-7}$ is hydroxy, when $R_1$-$R_3$ and $R_8$-$R_{12}$ are different from hydroxy; and
- $R_8$, $R_9$, $R_{10}$, $R_{11}$, and $R_{12}$ are independently selected from the group consisting of hydrogen, linear or branched $C_1$-$C_5$ alkoxy, alkyl linear or branched $C_1$-$C_6$, halogen, hydroxy, where at least one of $R_8$-$R_{12}$ is hydroxy, while $R_1$-$R_7$ are different from hydroxy.

In an implementation of the fifth embodiment, said catalyst of step (b) is selected from concentrated hydrochloric acid, acetic acid, trifluoroacetic acid, formic acid, or combinations thereof, and the solvent is selected from dimethylformamide, alcohols, or combinations thereof.

The compound(s) of Formula (I) of this invention have been prepared from the synthetic route described in General Scheme 1. However, those skilled in the art will readily notice that additional detailing and/or modifications to the arrangement of one or more steps can be accomplished without departing from the processes taught herein. Such variations can be, without limitation, combinations of solvents and catalysts, including stereoselective ones, protective groups, among others.

In the following General Scheme 1, the formation of the Formula II, III and IV intermediates is described, in addition to the formation of the Formula (I) compound(s).

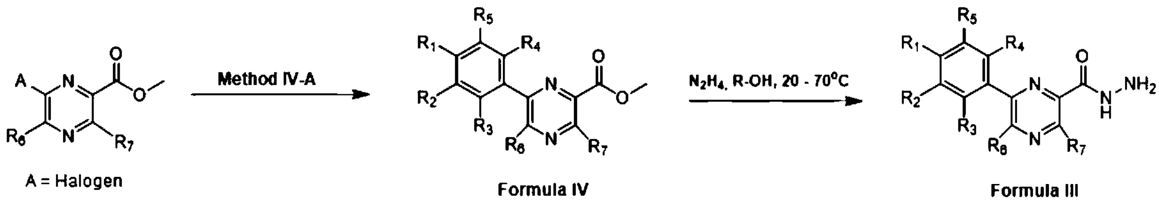


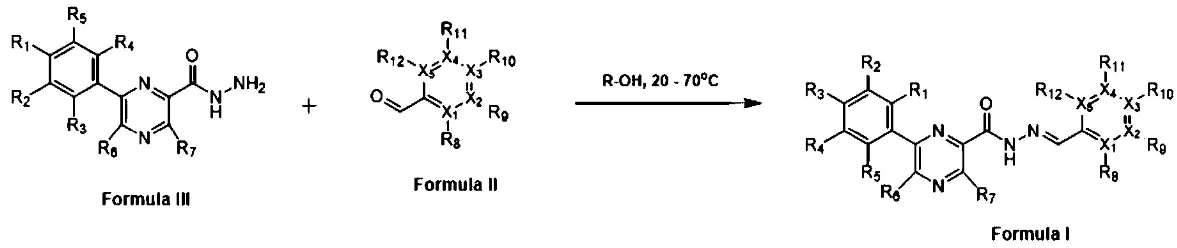


GENERAL SCHEME 1

**FORMULA IV INTERMEDIATES:**

Following the General Scheme 1, the method for the formation of the formula IV intermediates is presented, however such methods are not limiting.

**METHOD IV-A**

**INTERMEDIATES IV-1 TO IV-8:**

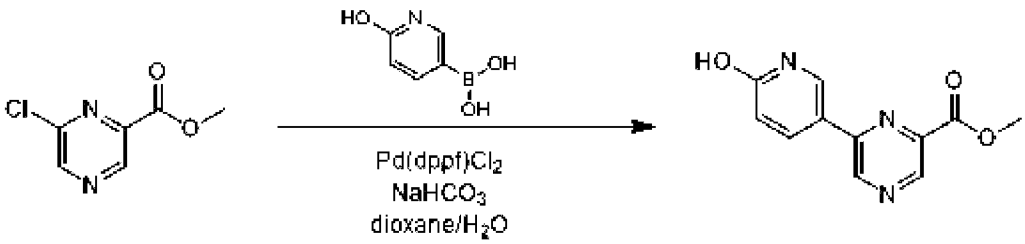


In a round-bottomed flask containing methyl 6-chloropyrazine-2-carboxylate (11.6 mmol) in dioxane (50.0 mL) and $H_2O$ (10.0 mL), (6-hydroxypyridine-3-yl)boronic acid (12.8 mmol), $NaHCO_3$ (17.4 mmol), and $Pd(dppf)Cl_2$ (579 μmol) were added under $N_2$ atmosphere. The reaction mixture was kept under agitation at 70 °C for 12 hours. After full consumption of the starting reagent (monitored by CCD), the mixture was concentrated under reduced pressure to remove the solvent. The residue obtained was diluted with $H_2O$ (100 mL) and extracted with AcOEt (100 mL x 3). The organic phase was concentrated under reduced pressure and purified by column chromatography (petroleum ether/AcOEt: 50/1 → 0/1). 1.5 g (56% yield) of the corresponding ester were obtained in the form of a white solid.

**TABLE 1. INTERMEDIATES OBTAINED FROM THE CORRESPONDING REACTANTS FOLLOWING THE METHOD DESCRIBED IN OBTAINING FORMULA IV INTERMEDIATES.**

| Intermediat e | Structure | Intermediate | Structure |
|---|---|---|---|
| IV-1 | 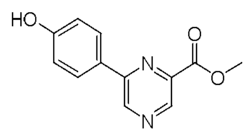 | IV-5 | 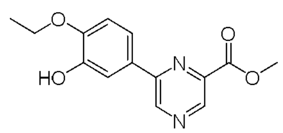 |

(continued)

| Intermediat e | Structure | Intermediate | Structure |
|---|---|---|---|
| IV-2 | 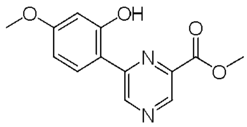 | IV-6 | 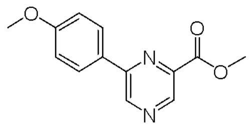 |
| IV-3 | 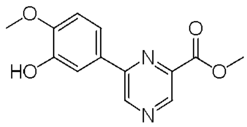 | IV-7 | 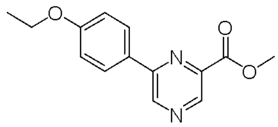 |
| IV-4 | 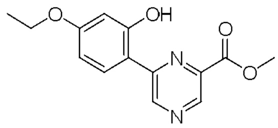 | IV-8 | 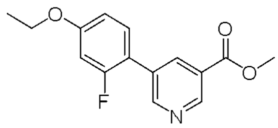 |

**OBTAINING THE FORMULA III INTERMEDIATES:**

Following General Scheme 1, the formula III intermediates are presented.

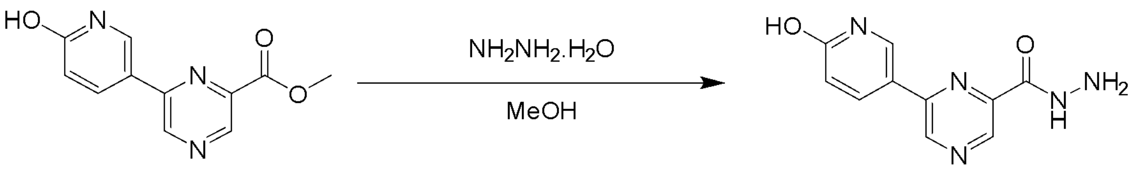


In a round-bottomed flask containing methyl 6-(6-hydroxypyridine-3-yl)pyrazine-2-carboxylate (11.6 mmol) in MeOH (30.0 mL), $N_2H_4 \cdot H_2O$ (17.4 mmol) was added. The reaction mixture was kept under agitation at 60 °C for 3 hours. After total consumption of the starting reagent (monitored by CCD), the mixture was filtered and concentrated. 1.6 g (60.0% yield) of the hydrazide of interest were obtained in the form of a white solid, which was sent to the next step without further purification (Table 2).

**TABLE 2. INTERMEDIATES OBTAINED FROM THE CORRESPONDING REAGENTS FOLLOWING THE METHOD DESCRIBED IN OBTAINING THE COMPOUNDS OF FORMULA III.**

| Intermediat e | Structure | Intermediate | Structure |
|---|---|---|---|
| III-1 | 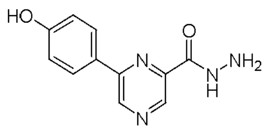 | III-7 | 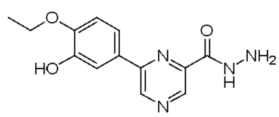 |
| III-2 | 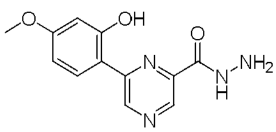 | III-8 | 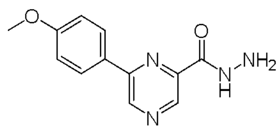 |
| III-3 | 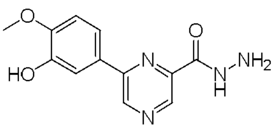 | III-9 | 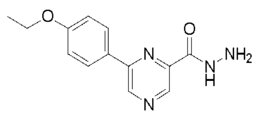 |
| III-4 | 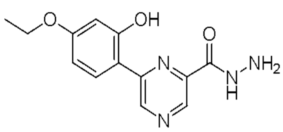 | III-10 | 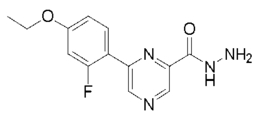 |

(continued)

| Intermediat e | Structure | Intermediate | Structure |
|---|---|---|---|
| III-5 | 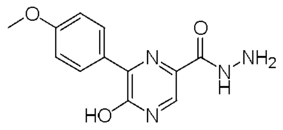 | III-11 | 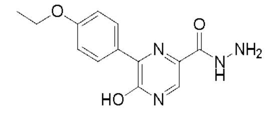 |
| III-6 | 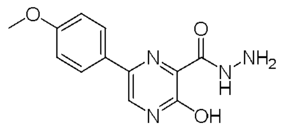 | III-12 | 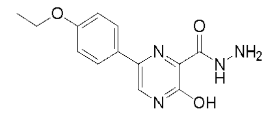 |
| III-13 | 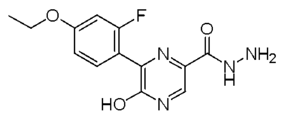 | III-14 | 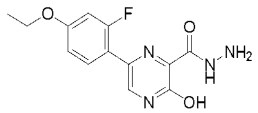 |

**OBTAINING FORMULA II COMPOUNDS**

Following General Scheme 1, there are now presented methods for the formation of Formula II intermediates without limitations.

**METHOD II-A**

**STEP II-A-I:**

**PRECURSOR II-1I:**

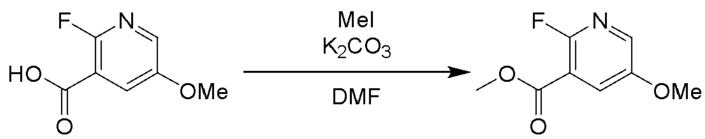


In a round-bottomed flask containing 2-fluoro-5-methoxynicotinic acid (64.3 mmol) and $K_2CO_3$ (96.4 mmol) in DMF (50.0 mL), iodomethane (129 mmol) were added. Subsequently, the reaction mixture was kept in agitation at 30 °C for 16 hours. After total consumption of the starting reagent (monitored by LC-MS), the reaction mixture was diluted with $H_2O$ (200 mL) and extracted with AcOEt (50.0 mL x 3). The organic phase was washed with saturated sodium chloride solution (*brine*) (100 mL x 2), dried on anhydrous sodium sulfate, filtered, concentrated under reduced pressure. 10.0 g (84.0% yield) of the product of interest was obtained in the form of a brown solid, which was used in the next step without further purification.

**STEP II-A-II:**

**PRECURSOR II-1II:**

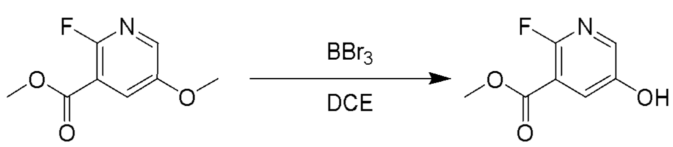


In a round-bottomed flask containing methyl 2-fluoro-5-methoxynicotinate (91.8 mmol) in DCE (400 mL), $BBr_3$ (230 mmol) was added at 0 °C. Subsequently, the reaction mixture was kept in agitation at 80 °C for 4 hours. After total

consumption of the starting reagent (monitored by LC-MS), the reaction mixture was cooled to 20 °C, diluted with $H_2O$ (200 mL) and extracted with DCM/MeOH: 5/1 (100 mL x 5). The organic phase was washed with saturated sodium chloride solution (*brine*) (100 mL), dried on anhydrous sodium sulfate, filtered and concentrated under reduced pressure. 11.0 g (70.0% yield) of the product of interest were obtained in the form of a brown solid, which was used for the next step without further purification.

**STEP II-A-II:**

**PRECURSOR II-1II:**

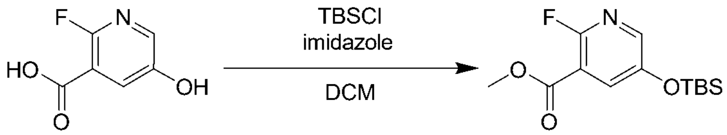


TBSCl (45.0 mmol) at 0 °C was added to a round-bottomed flask containing 2-fluoro-5-hydroxynicotinic acid (40.9 mmol) and imidazole (81.8 mmol) in DCM (50.0 mL). Subsequently, the reaction mixture was kept in agitation at 20 °C for 16 hours. After total consumption of the starting reagent (monitored by CCD), the reaction mixture was diluted with $H_2O$ (50.0 mL) and extracted with DCM (50.0 mL x 3). Subsequently, the organic phase was concentrated under reduced pressure and purified by column chromatography (petroleum ether/AcOEt: 50/1 $\rightarrow$ 0/1). 6.50 g (55.7% yield) of the product of interest were obtained in the form of a colorless oil.

**STEP II-A-III:**

**PRECURSOR II-1III:**

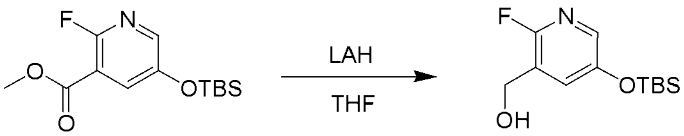


In a round-bottomed flask containing methyl 5-((tert-butyldimethylsilyl)oxy)-2-fluoronicotinate (22.8 mmol) in THF (50.0 mL), $LiAlH_4$ (45.6 mmol) was added at 0 °C. Subsequently, the reaction mixture was kept in agitation at 20 °C for 1 hour. After full consumption of the starting reagent (monitored by CCD), $Na_2SO_4 \cdot 10H$ was added to the reaction mixture $_2O$ (3.00 g). Next, the mixture was filtered and extracted with AcOEt (10.0 mL x 3). The organic phase was concentrated under reduced pressure. 4.00 g (68.2% yield) of the product of interest were obtained in the form of a yellow oil, which was used for the next step without further purification.

**STEP II-A-III:**

**PRECURSOR II-1III:**

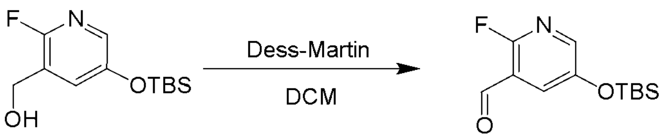


In a round-bottomed flask containing (5-((tert-butyldimethylsilyl)oxy)-2-fluoropyridin-3-yl)methanol (15.5 mmol) in DCM (30.0 mL), 1,1-diacetoxy-3-oxo-1,2-benziodoxol-1-yla acetate (23.3 mmol) was added at 0 °C. Subsequently, the reaction mixture was kept in agitation at 20 °C for 16 hours. After total consumption of the starting reagent (monitored by LC-MS and CCD), a solution of $NaHCO_3$ was added to the reaction mixture up to pH = 8. Next, the mixture was filtered,

diluted with $H_2O$ (50.0 mL) and extracted with DCM (50.0 mL x 3). The organic phase was concentrated under reduced pressure and purified by column chromatography (petroleum ether/AcOEt: 10/1 → 0/1). 3.20 g (80.6% yield) of the product of interest were obtained in the form of a yellow oil.

**STEP II-A-IV:**

**INTERMEDIATE II-1:**

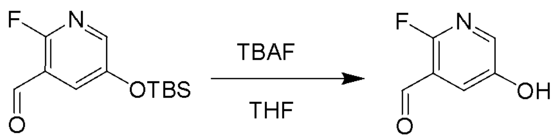


In a round-bottomed flask containing 5-((tert-butyldimethylsilyl)oxy)-2-fluoronicotinaldehyde (12.5 mmol) in THF (15.0 mL), TBAF (1.00 M, 18.8 mL) were added. Subsequently, the reaction mixture was kept in agitation at 20 °C for 1 hour. After total consumption of the starting reagent (monitored by LC-MS and CCD), the reaction mixture was diluted with $H_2O$ (10.0 mL) and extracted with AcOEt (10.0 mL x 3). Subsequently, the organic phase was washed with saturated sodium chloride solution (*brine*) (10.0 mL), dried on anhydrous sodium sulfate, filtered and concentrated under reduced pressure. The resulting product was purified by column chromatography (petroleum ether/AcOEt: 10/1 → 0/1). 1.50 g (84.8% yield) of the product of interest were obtained in the form of a white solid.

**METHOD II-B**

**STEP II-B-I:**

**INTERMEDIATE II-2:**

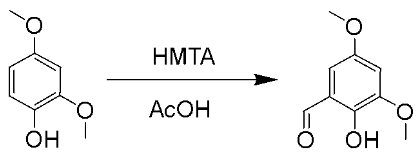


In a round-bottomed flask containing 2,4-dimethoxyphenol (25.95 mmol) in AcOH (40 mL), HMTA (51.89 mmol) were added. Subsequently, the reaction mixture was kept in agitation at 95 °C for 6 hours. After total consumption of the starting reagent (monitored by CCD), the reaction mixture was diluted with $H_2O$ (100 mL), an HCl solution was added (1N, 10 mL), and the mixture was left under agitation for 1 hour. Subsequently, the mixture was extracted with AcOEt (300 mL) and the organic phase was dried on anhydrous sodium sulfate, filtered and concentrated under reduced pressure. The resulting product was purified by column chromatography (petroleum ether/AcOEt: 30/1 → 10/1). 456 mg (9.65% yield) of the product of interest were obtained in the form of a yellow solid.

**EXAMPLES**

The following examples, described in detail, serve to illustrate the embodiments of this invention without, however, having any limitation character to the scope of protection thereof.

The General Formula I compound(s) of this invention were obtained from the condensation of Formula III intermediates with commercial aldehydes or with the Formula II intermediates described above, synthesized according to the various methodologies previously described and schematized in General Scheme 1, but not limited to these.

Compounds **1** to **36,** as shown in Table 3, are obtained by the procedure described for Example 1 (Compound 1), changing the corresponding intermediates II and III.

**EXAMPLE 1. (E)-N'-(3,5-DIMETHOXYBENZYLIDENE)-6-(6-HYDROXYPYRIDINE-3-IL)PYRAZINE-2-CARBOHYDRAZIDE**

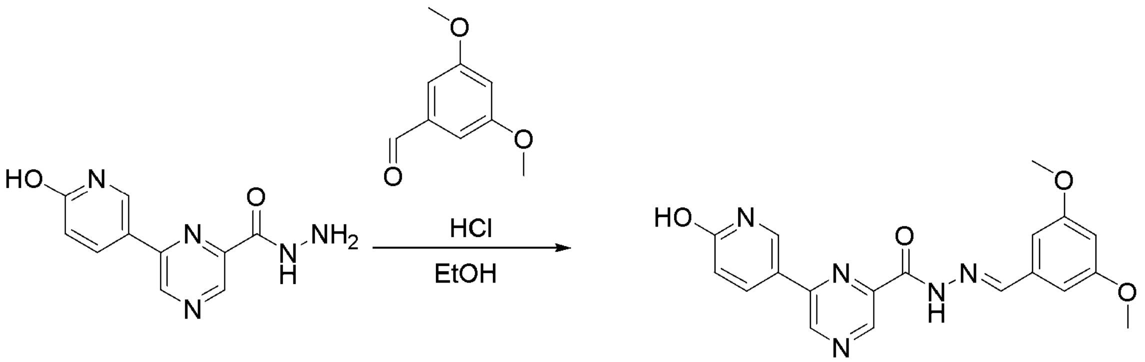


In a round-bottomed flask, 6-(6-hydroxypyridine-3-yl)pyrazine-2-carbohydrazide (2.08 mmol) were added to an aqueous solution of HCl (20.81 mmol). The reaction mixture was kept under agitation for 16 hours. After the reaction was finished, the mixture was concentrated under reduced pressure. The solid obtained was washed with hexane (20 mL x 3) and $H_2O$ (20 mL x 3), to obtain 540 mg (64% yield) of the product of interest **1** in the form of a white solid.

**TABLE 3. COMPOUNDS OBTAINED FROM THE CORRESPONDING REACTANTS FOLLOWING THE I-A METHOD.**

| Compound № | Structure | $^1$H-NMR / MS (m/z) $[M+H]^+$ |
|---|---|---|
| 1 | 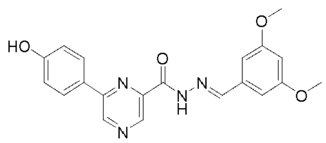 (*E*)-*N'*-(3,5-dimethoxybenzylidene)-6-(4-hydroxyphenyl) pyrazine-2-carbohydrazide | $^1$H-NMR, 400 MHz, DMSO-$d_6$ $\delta$ = 12.03 (br s, 1H), 10.18 - 9.87 (m, 1H), 9.41 (s, 1H), 9.07 (s, 1H), 8.66 (s, 1H), 8.31 (d, J = 8.8 Hz, 2H), 7.04 - 6.89 (m, 4H), 6.62 (t, J = 2.3 Hz, 1H), 3.82 (s, 6H). $[M+H]^+$: 379.1. |
| 2 | 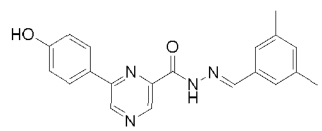 (*E*)-*N'*-(3,5-dimethylbenzylidene)-6-(4-hydroxyphenyl) pyrazine-2-carbohydrazide | $^1$H-NMR, 400 MHz, DMSO-$d_6$ $\delta$ = 11.99 (br s, 1H), 10.05 (br s, 1H), 9.41 (s, 1H), 9.07 (s, 1H), 8.64 (s, 1H), 8.31 (br d, J = 8.7 Hz, 2H), 7.40 (s, 2H), 7.12 (s, 1H), 6.96 (br d, J = 8.7 Hz, 2H), 2.35 (s, 6H). $[M+H]^+$: 347.1. |
| 3 | 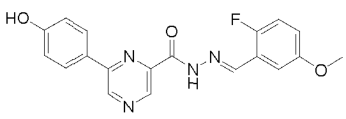 (*E*)-*N'*-(2-fluoro-5-methoxybenzylidene)-6-(4-hydroxyphenyl)pyrazine-2-carbohydrazide | $^1$H-NMR, 400 MHz, DMSO-$d_6$ $\delta$ = 12.21 (s, 1H), 10.05 (br s, 1H), 9.42 (s, 1H), 9.08 (s, 1H), 8.98 (s, 1H), 8.32 (d, J = 8.7 Hz, 2H), 7.44 (dd, J = 3.2, 5.5 Hz, 1H), 7.29 (t, J = 9.5 Hz, 1H), 7.10 (td, J = 3.8, 8.9 Hz, 1H), 6.97 (d, J = 8.8 Hz, 2H), 3.83 (s, 3H). $[M+H]^+$: 367.1. |
| 4 | 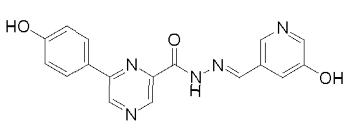 (*E*)-6-(4-hydroxyphenyl)-*N'*-((5-hydroxypyridine-3-yl) methylene)pyrazine-2-carbohydrazide | $^1$H-NMR, 400 MHz, DMSO-$d_6$ $\delta$ = 6.96 (d, J=8.76 Hz, 2 H) 7.57 - 7.62 (m, 1 H) 8.21 (d, J=2.75 Hz, 1 H) 8.27 - 8.35 (m, 3 H) 8.72 (s, 1 H) 9.07 (s, 1 H) 9.41 (s, 1 H) 9.94 - 10.33 (m, 2 H) 12.14 (s, 1 H). $[M+H]^+$: 336.4. |

(continued)

| Compound № | Structure | $^1$H-NMR / MS (m/z) [M+H]$^+$ |
|---|---|---|
| 5 | 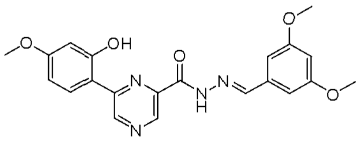 (*E*)-*N*'-(3,5-dimethoxybenzylidene)-6-(2-hydroxy-4-methoxyphenyl)pyrazine-2-carbohydrazide | $^1$H-NMR, 400 MHz, DMSO-$d_6$ $\delta$ = 12.06 (s, 1H), 10.84 (s, 1H), 9.51 (s, 1H), 9.04 (s, 1H), 8.60 (s, 1H), 8.24 (d, J = 8.7 Hz, 1H), 6.93 (d, J = 2.1 Hz, 2H), 6.71 - 6.53 (m, 3H), 3.82 (d, J = 1.6 Hz, 9H). [M+H]$^+$: 409,1. |
| 6 | 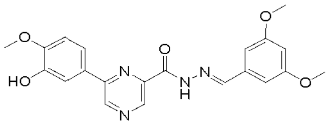 (*E*)-*N'*-(3,5-dimethoxybenzylidene)-6-(3-hydroxy-4-methoxyphenyl)pyrazine-2-carbohydrazide | $^1$H-NMR, 400 MHz, DMSO-$d_6$ $\delta$ = 12.10 (br s, 1H), 9.38 (s, 1H), 9.23 (br s, 1H), 9.08 (s, 1H), 8.64 (s, 1H), 7.91 - 7.81 (m, 2H), 7.11 (d, J = 8.8 Hz, 1H), 6.93 (d, J = 2.2 Hz, 2H), 6.62 (t, J = 2.1 Hz, 1H), 3.89 (s, 3H), 3.82 (s, 6H). [M+H]$^+$: 409.1. |
| 7 | 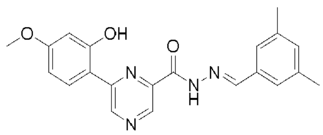 (*E*)-*N*'-(3,5-dimethylbenzylidene)-6-(2-hydroxy-4-methoxyphenyl)pyrazine-2-carbohydrazide | $^1$H-NMR, 400 MHz, DMSO-$d_6$ $\delta$ = 12.02 (s, 1H), 10.85 (s, 1H), 9.50 (s, 1H), 9.04 (s, 1H), 8.58 (s, 1H), 8.22 (br d, J = 8.7 Hz, 1H), 7.39 (s, 2H), 7.17 - 7.08 (m, 1H), 6.72 - 6.55 (m, 2H), 3.81 (s, 3H), 2.34 (s, 6H). [M+H]$^+$: 377.1. |
| 8 | 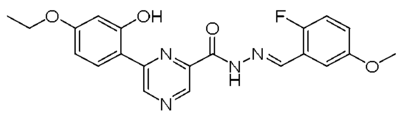 (*E*)-6-(4-ethoxy-2-hydroxyphenyl)-*N'*-(2-fluoro-5-methoxybenzylidene)pyraz ine-2-carbohydrazide | $^1$H-NMR, 400 MHz, DMSO-$d_6$ $\delta$ = 12.23 (s, 1H), 10.78 (s, 1H), 9.52 (s, 1H), 9.04 (s, 1H), 8.92 (s, 1H), 8.23 (d, J = 8.8 Hz, 1H), 7.43 (dd, J = 3.3, 5.6 Hz, 1H), 7.28 (t, J = 9.6 Hz, 1H), 7.09 (td, J = 3.8, 8.8 Hz, 1H), 6.69 - 6.54 (m, 2H), 4.08 (q, J = 7.0 Hz, 2H), 3.83 (s, 3H), 1.36 (t, J = 6.9 Hz, 3H). [M+H]$^+$: 411.4. |
| 9 | 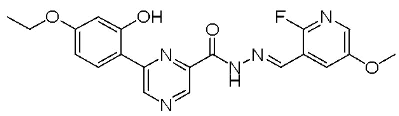 (*E*)-6-(4-ethoxy-2-hydroxyphenyl)-*N'*-((2-fluoro-5-methoxypyridine-3-yl)methylene)pyrazine-2-carbohydrazide | $^1$H-NMR, 400 MHz, DMSO-$d_6$ $\delta$ = 12.35 (br s, 1H), 10.85 - 10.64 (m, 1H), 9.54 - 9.49 (m, 1H), 9.04 (s, 1H), 8.86 - 8.82 (m, 1H), 8.21 (d, J = 8.8 Hz, 1H), 8.02 (dd, J = 1.8, 3.0 Hz, 1H), 7.90 (dd, J = 3.1, 7.6 Hz, 1H), 6.67 - 6.61 (m, 1H), 6.57 (d, J = 2.4 Hz, 1H), 4.07 (q, J = 7.0 Hz, 2H), 3.91 (s, 3H), 1.36 (t, J = 6.9 Hz, 3H). [M+H]$^+$: 412.0. |
| 10 | 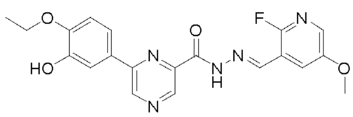 (*E*)-6-(4-ethoxy-3-hydroxyphenyl)-*N'*-((2-fluoro-5-methoxypyridine-3-yl)methylene)pyrazine-2-carbohydrazide | |
| 11 | 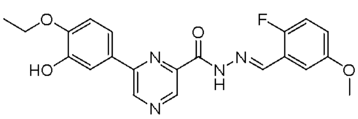 (*E*)-6-(4-ethoxy-3-hydroxyphenyl)-*N'*-(2-fluoro-5-methoxybenzylidene)pyrazine-2-carbohydrazide | |

(continued)

| Compound № | Structure | $^1$H-NMR / MS (m/z) $[M+H]^+$ |
|---|---|---|
| 12 | 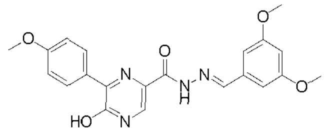 (*E*)-*N*'-(3.5-dimethoxybenzylidene)-5-hydroxy-6-(4-methoxyphenyl)pyrazine-2-carbohydrazide | $^1$H-NMR, 400 MHz, DMSO-$d_6$ $\delta$ = 13.07 (s, 1H) , 11.48 (br d, J = 0.7 Hz, 1H) , 8.63 - 8.44 (m, 3H), 7.99 (s, 1H) , 7.01 (br d, J = 9.0 Hz, 2H) , 6.87 (br d, J = 1.8 Hz, 2H) , 6.59 - 6.53 (m, 1H) , 3.82 (s, 3H) , 3.78 (s, 6H). $[M+H]^+$: 409.1. |
| 13 | 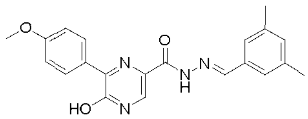 (*E*)-*N'*-(3.5-dimethylbenzylidene)-5-hydroxy-6-(4-methoxyphenyl)pyrazine-2-carbohydrazide | $^1$H-NMR, 400 MHz, DMSO-$d_6$ $\delta$ = 13.02 - 12.75 (m, 1H), 11.46 (s, 1H), 8.66 - 8.51 (m, 3H), 8.02 (s, 1H), 7.36 (s, 2H), 7.13 - 7.01 (m, 3H), 3.85 (s, 3H), 2.33 (s, 6H). $[M+H]^+$: 377.1. |
| 14 | 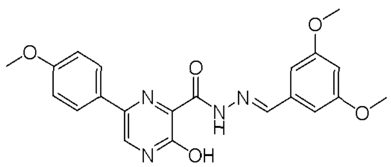 (*E*)-*N*'-(3.5-dimethoxybenzylidene)-3-hydroxy-6-(4-methoxyphenyl)pyrazine-2-carbohydrazide | $^1$H-NMR, 400 MHz, DMSO-$d_6$ $\delta$ = 8.48 (br d, J = 6.7 Hz, 2H) , 8.08 - 7.61 (m, 2H), 7.10 - 6.89 (m, 4H), 6.73 - 6.31 (m, 2H) , 3.86 - 3.74 (m, 9H). $[M+H]^+$: 409.1. |
| 15 | 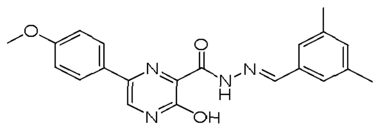 (*E*)-*N*'-(3.5-dimethylbenzylidene)-3-hydroxy-6-(4-methoxyphenyl)pyrazine-2-carbohydrazide | $^1$H-NMR, 400 MHz, DMSO-$d_6$ $\delta$ = 8.58 - 8.22 (m, 2H) , 7.94 (br d, J = 7.3 Hz, 2H), 7.39 (s, 2H) , 7.18 - 6.87 (m, 3H) , 3.83 - 3.78 (m, 3H), 2.34 (s, 5H). $[M+H]^+$: 377.1. |
| 16 | 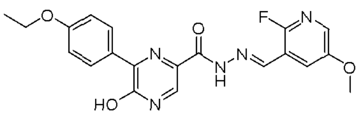 (*E*)-6-(4-ethoxyphenyl)-*N'*-((2-fluoro-5-methoxypyridine-3-yl)methylene)-5-hydroxypyrazine-2-carbohydrazide | $^1$H-NMR, 400 MHz, DMSO-$d_6$ $\delta$ = 13.05 - 12.74 (m, 1H), 11.85 (s, 1H), 8.82 (s, 1H), 8.63 (d, J = 1.4 Hz, 2H), 8.05 - 7.98 (m, 2H), 7.87 (dd, J = 3.0, 7.8 Hz, 1H), 7.03 (d, J = 9.0 Hz, 2H), 4.12 (d, J = 7.0 Hz, 2H), 3.91 (s, 3H), 1.37 (t, J = 7.0 Hz, 3H). $[M+H]^+$: 412.0. |
| 17 | 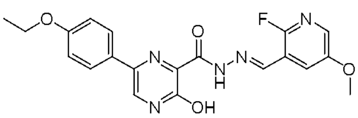 (*E*)-6-(4-ethoxyphenyl)-*N'*-((2-fluoro-5-methoxypyridine-3-yl)methylene)-3-hydroxypyrazine-2-carbohydrazide | |
| 18 | 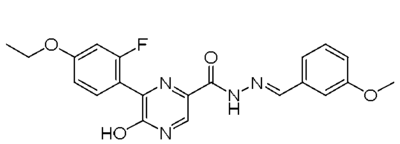 (*E*)-6-(4-ethoxy-2-fluorophenyl)-5-hydroxy-*N'*-(*3*-methoxybenzylidene)pyraz ine-2-carbohydrazide | $^1$H-NMR, 400 MHz, DMSO-$d_6$ $\delta$ = 12.96 (td, J = 1.5, 2.8 Hz, 1H), 11.55 - 10.73 (m, 1H), 8.51 (s, 1H), 8.09 (br s, 1H), 7.65 (br t, J = 8.5 Hz, 1H), 7.43 - 7.33 (m, 1H), 7.29 - 7.20 (m, 2H), 7.01 (br d, J = 8.8 Hz, 1H), 6.94 - 6.86 (m, 2H), 4.12 (q, J = 6.9 Hz, 2H), 3.82 - 3.76 (m, 3H), 1.36 (br t, J = 6.8 Hz, 3H). $[M+H]^+$: 411.1. |

(continued)

| Compoun d № | Structure | $^1$H-NMR / MS (m/z) $[M+H]^+$ |
|---|---|---|
| 19 | 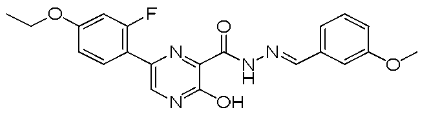<br>(*E*)-6-(4-ethoxy-2-fluorophenyl)-3-hydroxy-*N'*-(3-methoxybenzylidene)pyraz ine-2-carbohydrazide | $^1$H-NMR, 400 MHz, DMSO-$d_6$ $\delta$ = 12.44 - 12.09 (m, 1H), 8.44 (s, 1H), 8.00 - 7.88 (m, 1H), 7.72 (t, J = 9.2 Hz, 1H), 7.52 - 7.21 (m, 3H), 7.11 - 6.81 (m, 4H), 4.09 (quin, J = 6.8 Hz, 2H), 3.83 - 3.66 (m, 3H), 1.38 - 1.30 (m, 3H).<br>$[M+H]^+$: 411.2. |
| 20 | 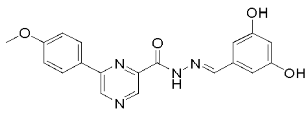<br>(*E*)-*N'*-(3,5-dihydroxybenzylidene)-6-(4-methoxyphenyl) pyrazine-2-carbohydrazide | $^1$H-NMR, 400 MHz, DMSO-$d_6$ $\delta$ = 11.95 (s, 1H), 9.49 (d, J = 15.9 Hz, 3H), 9.11 (s, 1H), 8.53 (s, 1H), 8.42 (d, J = 8.8 Hz, 2H), 7.15 (d, J = 8.9 Hz, 2H), 6.67 (d, J = 2.2 Hz, 2H), 6.30 (t, J = 2.1 Hz, 1H), 3.88 (s, 3H).<br>$[M+H]^+$: 365.0. |
| 21 | 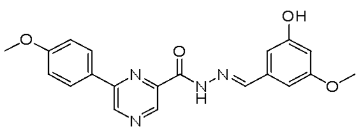<br>(*E*)-*N'*-(3-hydroxy-5-methoxybenzylidene)-6-(4-methoxyphenyl)pyrazine-2-carbohydrazide | $^1$H-NMR, 400 MHz, DMSO-$d_6$ $\delta$ = 12.01 (br s, 1H), 9.73 (br s, 1H), 9.47 (s, 1H), 9.11 (s, 1H), 8.59 (s, 1H), 8.48 - 8.37 (m, 2H), 7.19 - 7.11 (m, 2H), 6.85 (d, J = 1.4 Hz, 1H), 6.76 (s, 1H), 6.44 (t, J = 2.2 Hz, 1H), 3.88 (s, 3H), 3.77 (s, 3H).<br>$[M+H]^+$: 379.1. |
| 22 | 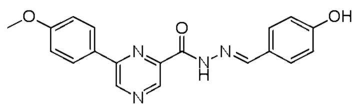<br>(*E*)-*N'*-(4-hydroxybenzylidene)-6-(4-methoxyphenyl)pyrazine-2-carbohydrazide | $^1$H-NMR, 400 MHz, DMSO-$d_6$ $\delta$ = 11.88 (s, 1H), 10.00 (s, 1H), 9.46 (s, 1H), 9.10 (s, 1H), 8.61 (s, 1H), 8.42 (d, J = 8.9 Hz, 2H), 7.63 (d, J = 8.7 Hz, 2H), 7.14 (d, J = 8.9 Hz, 2H), 6.88 (d, J = 8.6 Hz, 2H), 3.88 (s, 3H)<br>$[M+H]^+$: 349.1. |
| 23 | 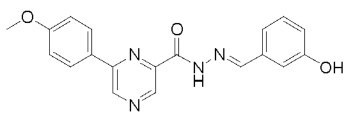<br>(*E*)-*N'*-(3-hydroxybenzylidene)-6-(4-methoxyphenyl)pyrazine-2-carbohydrazide | $^1$H-NMR, 400 MHz, DMSO-$d_6$ $\delta$ = 12.01 (br s, 1H), 9.69 (br s, 1H), 9.47 (s, 1H), 9.11 (s, 1H), 8.64 (s, 1H), 8.42 (d, J = 8.8 Hz, 2H), 7.34 - 7.24 (m, 2H), 7.21 - 7.10 (m, 3H), 6.87 (dd, J = 2.1, 7.8 Hz, 1H), 3.88 (s, 3H).<br>$[M+H]^+$: 349.1. |
| 24 | 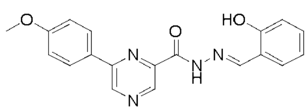<br>(*E*)-*N'*-(2-hydroxybenzylidene)-6-(4-methoxyphenyl)pyrazine-2-carbohydrazide | $^1$H-NMR, 400 MHz, DMSO-$d_6$ $\delta$ = 12.33 (br s, 1H), 11.21 (br s, 1H), 9.48 (s, 1H), 9.13 (s, 1H), 8.95 (s, 1H), 8.49 - 8.38 (m, 2H), 7.61 (dd, J = 1.5, 7.7 Hz, 1H), 7.39 - 7.30 (m, 1H), 7.16 (d, J = 8.8 Hz, 2H), 7.03 - 6.91 (m, 2H), 3.88 (s, 3H).<br>$[M+H]^+$: 349.1. |
| 25 | 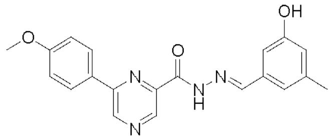<br>(*E*)-*N'*-(3-hydroxy-5-methylbenzylidene)-6-(4-methoxyphenyl)pyrazine-2-carbohydrazide | $^1$H-NMR, 400 MHz, DMSO-$d_6$ $\delta$ = 11.99 (br s, 1H), 10.05 (br s, 1H), 9.41 (s, 1H), 9.07 (s, 1H), 8.64 (s, 1H), 8.31 (br d, J = 8.7 Hz, 2H), 7.40 (s, 2H), 7.12 (s, 1H), 6.96 (br d, J = 8.7 Hz, 2H), 2.35 (s, 6H).<br>$[M+H]^+$: 363.1. |

(continued)

| Compound № | Structure | $^1$H-NMR / MS (m/z) $[M+H]^+$ |
|---|---|---|
| 26 | 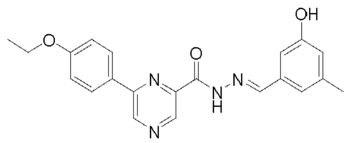 (*E*)-6-(4-ethoxyphenyl)-*N'*-(3-hydroxy-5-methylbenzylidene)pyrazi ne-2-carbohydrazide | $^1$H-NMR, 400 MHz, DMSO-$d_6$ $\delta$ = 11.99 (s, 1H), 9.56 (s, 1H), 9.46 (s, 1H), 9.10 (s, 1H), 8.59 (s, 1H), 8.40 (d, J = 8.8 Hz, 2H), 7.13 (d, J = 8.9 Hz, 2H), 7.02 (d, J = 16.1 Hz, 2H), 6.69 (s, 1H), 4.16 (q, J = 7.0 Hz, 2H), 2.29 (s, 3H), 1.39 (t, J = 6.9 Hz, 3H). $[M+H]^+$: 377.1. |
| 27 | 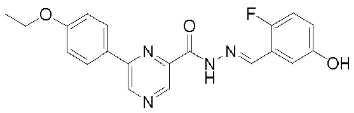 (*E*)-6-(4-ethoxyphenyl)-*N'*-(2-fluoro-5-hydroxybenzylidene)pyraz ine-2-carbohydrazide | $^1$H-NMR, 400 MHz, DMSO-$d_6$ $\delta$ = 12.22 (s, 1H), 9.68 (s, 1H), 9.48 (s, 1H), 9.12 (s, 1H), 8.92 (s, 1H), 8.42 (br d, J = 8.8 Hz, 2H), 7.41 (dd, J = 3.1, 5.7 Hz, 1H), 7.21 - 7.08 (m, 3H), 6.93 - 6.82 (m, 1H), 4.15 (q, J = 6.8 Hz, 2H), 1.39 (t, J = 7.0 Hz, 3H) . $[M+H]^+$: 381.1. |
| 28 | 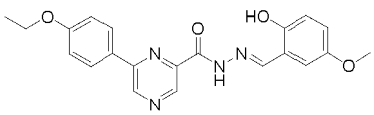 (*E*)-6-(4-ethoxyphenyl)-*N'*-(2-hydroxy-5-methoxybenzylidene)pyraz ine-2-carbohydrazide | $^1$H-NMR, 400 MHz, DMSO-$d_6$ $\delta$ = 12.28 (s, 1H), 10.59 (s, 1H), 9.46 (s, 1H), 9.10 (s, 1H), 8.92 (s, 1H), 8.41 (d, J = 8.9 Hz, 2H), 7.20 - 7.08 (m, 3H), 6.98 - 6.86 (m, 2H), 4.15 (q, J = 7.0 Hz, 2H), 3.75 (s, 3H), 1.38 (t, J = 6.9 Hz, 3H). $[M+H]^+$: 393.3. |
| 29 | 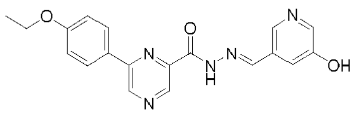 (*E*)-6-(4-ethoxyphenyl)-*N'*-((5-hydroxypyridine-3-yl) methylene)pyrazine-2-carbohydrazide | $^1$H-NMR, 400 MHz, DMSO-$d_6$ $\delta$ = 12.19 (s, 1H), 10.39 - 10.22 (m, 1H), 9.46 (s, 1H), 9.11 (s, 1H), 8.75 - 8.70 (m, 1H), 8.40 (d, J = 8.9 Hz, 2H), 8.35 (d, J = 1.6 Hz, 1H), 8.22 (d, J = 2.8 Hz, 1H), 7.72 - 7.56 (m, 1H), 7.12 (d, J = 8.9 Hz, 2H), 4.15 (q, J = 7.0 Hz, 2H), 1.38 (t, J = 6.9 Hz, 3H). $[M+H]^+$: 364.4. |
| 30 | 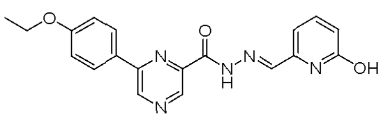 (*E*)-6-(4-ethoxyphenyl)-*N'*-((6-hydroxypyridine-2-yl) methylene)pyrazine-2-carbohydrazide | $^1$H-NMR, 400 MHz, DMSO-$d_6$ $\delta$ = 12.47 (s, 1H), 11.14 (brs, 1H), 9.47 (s, 1H), 9.11 (s, 1H), 8.53 (s, 1H), 8.40 (d, J = 8.8 Hz, 2H), 7.57 (t, J = 8 Hz, 1H), 7.11 (d, J = 8 Hz, 2H), 6.80 (brs, 1H), 6.51 (d, J = 8 Hz, 1H), 4.17-4.15, 4.17-4.11 (q, J= 8 Hz, 2H), 1.37 (t, J = 8 Hz, 3H). $[M+H]^+$: 362.1. |
| 31 | 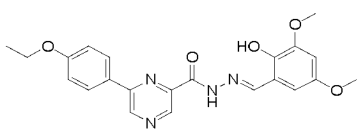 (*E*)-6-(4-ethoxyphenyl)-*N'*-(2-hydroxy-3,5-dimethoxybenzylidene)pyr azine-2-carbohydrazide | $^1$H-NMR, 400 MHz, DMSO-$d_6$ $\delta$ = 12.24 (br s, 1H), 10.08 (s, 1H), 9.46 (s, 1H), 9.10 (s, 1H), 8.95 (s, 1H), 8.41 (d, J = 8.8 Hz, 2H), 7.17 - 7.08 (m, 2H), 6.79 - 6.64 (m, 2H), 4.14 (q, J = 7.0 Hz, 2H), 3.87 - 3.70 (m, 6H), 1.38 (t, J = 7.0 Hz, 3H). $[M+H]^+$: 423.2. |

(continued)

| Compound № | Structure | $^1$H-NMR / MS (m/z) [M+H]$^+$ |
|---|---|---|
| 32 | 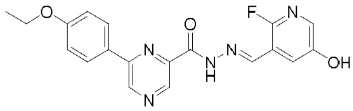 (*E*)-6-(4-ethoxyphenyl)-*N'*-((2-fluoro-5-hydroxypyridine-3-yl)methylene)pyrazine-2-carbohydrazide | $^1$H-NMR, 400 MHz, DMSO-$d_6$ $\delta$ = 12.32 (s, 1H), 10.25 (s, 1H), 9.47 (s, 1H), 9.11 (s, 1H), 8.84 (s, 1H), 8.40 (d, J = 8.9 Hz, 2H), 7.90 - 7.74 (m, 2H), 7.12 (d, J = 8.8 Hz, 2H), 4.14 (q, J = 6.9 Hz, 2H), 1.38 (t, J = 6.9 Hz, 3H) . [M+H]$^+$: 382.4. |
| 33 | 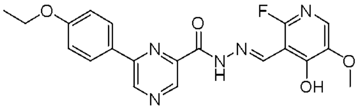 (*E*)-6-(4-ethoxyphenyl)-*N'*-((2-fluoro-4-hydroxy-5-methoxypyridine-3-yl)methylene)pyrazine-2-carbohydrazide | |
| 34 | 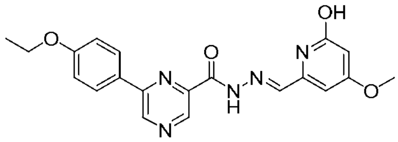 (*E*)-6-(4-ethoxyphenyl)-*N'*-((6-hydroxy-4-methoxypyridine-2-yl)methylene)pyrazine-2-carbohydrazide | |
| 35 | 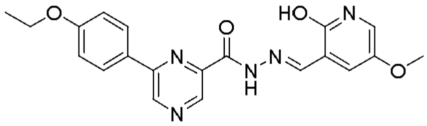 (*E*)-6-(4-ethoxyphenyl)-*N'*-((2-hydroxy-5-methoxypyridine-3-yl)methylene)pyrazine-2-carbohydrazide | |
| 36 | 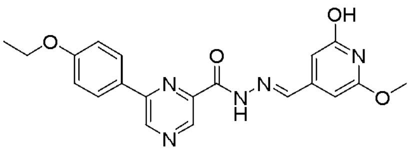 (*E*)-6-(4-ethoxyphenyl)-*N'*-((2-hydroxy-6-methoxypyridine-4-yl)methylene)pyrazine-2-carbohydrazide | |

**EXAMPLE 3. TESTS *IN VITRO:***

Biological assays were performed on Chinese hamster ovary (CHO) cells that express human sodium channels Nav 1.8 or Nav 1.7 in a stable manner.

The experimental protocol *voltage-clamp* using the whole cell configuration was established on the automated ScreenPatch® 384P platform (SP384PE, Nanion Technologies, Livingston, NJ) and the results were recorded with a Nanion 384-well Patch Clamp chip (NPC) (Nanion Technologies, Livingston, NJ).

Formula I compounds were diluted in eight concentrations in the extracellular solution composed of physiological saline solution, buffered with HEPES (mM): NaCl, 137; KCl, 4; $CaCl_2$, 3.8; $MgCl_2$, 1; HEPES, 10; Glucose, 10; pH 7.4. The extracellular solution is composed of (mM) CsCl, 50; CsF, 90; $MgCl_2$, 5; EGTA, 5; HEPES, 10; pH 7.2. The duration of exposure of each compound with cells expressing Nav 1.7 or Nav 1.8 was at least five minutes, and the assays were performed at room temperature.

The measurements of the sodium currents of Nav 1.8 and Nav 1.7 were obtained using the voltage protocol described below.

A holding voltage of -100 mV was established followed by an inactivation voltage step at -40 mV for 8 seconds, followed by a step of -100 mV for 20 ms, followed by a 20 ms step for 10 mV for Nav 1.8 or 0 mV for Nav 1.7 (TP1A) before returning to the holding voltage of -100 mV.

The protocol was repeated at a frequency of 0.05 Hz and the amplitude of the current was quantified throughout

the recording of the TP1A phase. The variation in the peak current amplitude was evaluated according to the Formula described below, after exposing the cells expressing the channels to each concentration of the different compounds:

$$\% \text{ Block} = (1 - (I_{TP1A,molecule} / I_{TP1A,basal}) \times 100\%,$$

wherein $I_{TP1A,basal}$ and $I_{TP1A,molecule}$ represent the peaks of sodium current input into TP1A before exposure to the compound and in the presence of the compound, respectively.

The decrease in the peak current amplitude, after the exposure of the cells to the compounds, was used to calculate the percentage of relative blockage of the channels in relation to the positive control according to the Formula below:

$$\% \text{ Block'} = 100\% - ((\% \text{ Block} - \% \text{ CP}) * (100\% / (\%V - \% \text{ CP})),$$

where %V and %CP represent the means of the values of current inhibition with a vehicle (DMSO) and positive controls, respectively. The sodium currents of the positive control were considered as 100%:

$$\% \text{ Block'} = (100\% / [1 + ([\text{Test}] / CI_{50})^N],$$

wherein [Test] represents the concentration of the molecule evaluated, $IC_{50}$ is the concentration of the compound that generates half of the maximum inhibition, N is Hill's coefficient, and % Blockage' is the percentage of sodium channel current (Nav 1.8 and Nav 1.7) inhibited at each concentration of the molecule evaluated. Data were obtained by nonlinear regression *(nonlinear least squares)* with XLfit for Excel (Microsoft, Redmond, WA).

The compounds were tested in at least one assay to obtain the value of $CI_{50}$. For compounds that were tested in two or more assays, the results are described as the means of the $CI_{50}$ values.

Table 4 shows the efficacy *in vitro* of selected compounds against Nav1.8 and Nav1.7. $CI_{50}$ values less than 500 nM are represented with the legend (+++); $CI_{50}$ values between 500 nM and 1000 nM are represented with the legend (++), $CI_{50}$ values greater than 1000 nM are demonstrated with the symbology (+).

**TABLE 4. $CI_{50}$ VALUES OF THE COMPOUNDS OF THIS INVENTION IN NAV 1.8 AND NAV 1.7 CHANNELS.**

| Compound № | Nav1.8 $IC_{50}$ (nM) | Nav1.7 $IC_{50}$ (nM) |
|---|---|---|
| 1 | + | + |
| 2 | + | + |
| 3 | + | + |
| 4 | + | + |
| 5 | +++ | + |
| 6 | + | + |
| 7 | +++ | + |
| 8 | ++ | + |
| 9 | +++ | + |
| 12 | + | + |
| 13 | + | + |
| 14 | + | + |
| 15 | + | + |
| 16 | + | + |
| 18 | + | + |
| 19 | + | + |
| 20 | + | + |
| 21 | + | + |
| 22 | + | + |

(continued)

| Compound № | Nav1.8 $IC_{50}$ (nM) | Nav1.7 $IC_{50}$ (nM) |
|---|---|---|
| 23 | + | + |
| 24 | + | + |
| 25 | ++ | + |
| 26 | +++ | + |
| 27 | ++ | + |
| 28 | +++ | + |
| 29 | + | + |

From these results, the blocking activity of Nav 1.7 and/or 1.8 is proven, whose application is readily performed by those skilled in the art in pharmaceutical compositions, which may comprise one or more of the aforementioned Formula I compounds, kits, in addition to uses in the treatment of pain-related pathologies.

In particular, these results indicate the possibility of using Formula (I) compounds in the preparation of drugs for the treatment of conditions such as peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

It should be understood that the embodiments described above are merely illustrative and that several modifications can be made by a person skilled in the art without departing from the scope of this invention. Consequently, the present invention should not be regarded as being limited to the illustrative specifications described in this application.

## Claims

1. COMPOUND, **characterized by** being of Formula (I)

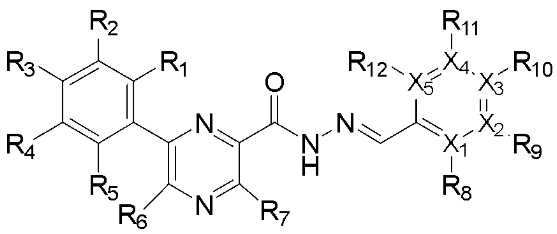

Formula (I)

or a pharmaceutically acceptable salt, hydrate, solvate and isomer thereof, wherein:

- one of $X_1$, $X_2$, $X_3$, $X_4$ and $X_5$ is nitrogen and the others are carbon, where when one of $X_1$, $X_2$, $X_3$, $X_4$ and $X_5$ is nitrogen, the corresponding R ($R_5$, $R_6$ and/or $R_7$) will be null;
- $R_1$, $R_2$, $R_3$, $R_4$, and $R_5$ are independently selected from the group consisting of hydrogen, halogen, hydroxy, alkoxy $C_1$-$C_6$ linear or branched, alkyl $C_1$-$C_5$ linear or branched, wherein at least one of $R_1$-$R_3$ is hydroxy, when $R_6$-$R_{12}$ are different from hydroxy;
- $R_6$ and $R_7$ are independently selected from the group consisting of hydrogen, halogen, hydroxy, linear or branched $C_1$-$C_6$ alkoxy, $C_1$-$C_6$ linear or branched alkyl, wherein at least one of $R_6$-$R_7$ is hydroxy, when $R_1$-$R_5$ and $R_8$-$R_{12}$ are different from hydroxy; and
- $R_8$, $R_9$, $R_{10}$, $R_{11}$, and $R_{12}$ are independently selected from the group consisting of hydrogen, linear or branched $C_1$-$C_6$ alkoxy, alkyl linear or branched $C_1$-$C_6$; halogen, hydroxy, wherein at least one of $R_8$-$R_{12}$ is hydroxy, while $R_1$-$R_7$ are different from hydroxy.

2. COMPOUND, according to claim 1, **characterized by** being selected from the group consisting of:

- (*E*)-*N'*-(3,5-dimethoxybenzylidene)-6-(4-hydroxyphenyl)pyrazine-2-carbohydrazide (Compound 1);
- (*E*)-*N'*-(3,5-dimethylbenzylidene)-6-(4-hydroxyphenyl)pyrazine-2-carbohydrazide (Compound 2);
- (*E*)-*N'*-(2-fluoro-5-methoxybenzylidene)-6-(4-hydroxyphenyl)pyrazine-2-carbohydrazide (Compound 3);

- (*E*)-6-(4-hydroxyphenyl)-*N'*-((5-hydroxypyridine-3-yl)methylene)pyrazine-2-carbohydrazide (Compound 4);
- (*E*)-*N'*-(3,5-dimethoxybenzylidene)-6-(2-hydroxy-4-methoxyphenyl)pyrazine-2-carbohydrazide (Compound 5);
- (*E*)-*N'*-(3,5-dimethoxybenzylidene)-6-(3-hydroxy-4-methoxyphenyl)pyrazine-2-carbohydrazide (Compound 6);
- (*E*)-*N'*-(3,5-dimethylbenzylidene)-6-(2-hydroxy-4-methoxyphenyl)pyrazine-2-carbohydrazide (Compound 7);
- (*E*)-6-(4-ethoxy-2-hydroxyphenyl)-*N'*-(2-fluoro-5-methoxybenzylidene)pyrazine-2-carbohydrazide (Compound 8);
- (*E*)-6-(4-ethoxy-2-hydroxyphenyl)-*N'*-((2-fluoro-5-methoxypyridine-3-yl)methylene)pyrazine-2-carbohydrazide (Compound 9);
- (*E*)-6-(4-ethoxy-3-hydroxyphenyl)-*N'*-((2-fluoro-5-methoxypyridine-3-yl)methylene)pyrazine-2-carbohydrazide (Compound 10);
- (*E*)-6-(4-ethoxy-3-hydroxyphenyl)-*N'*-(2-fluoro-5-methoxybenzylidene)pyrazine-2-carbohydrazide (Compound 11);
- (*E*)-*N'*-(3,5-dimethoxybenzylidene)-5-hydroxy-6-(4-methoxyphenyl)pyrazine-2-carbohydrazide (Compound 12);
- (*E*)-*N'*-(3,5-dimethylbenzylidene)-5-hydroxy-6-(4-methoxyphenyl)pyrazine-2-carbohydrazide (Compound 13);
- (*E*)-*N'*-(3,5-dimethoxybenzylidene)-3-hydroxy-6-(4-methoxyphenyl)pyrazine-2-carbohydrazide (Compound 14);
- (*E*)-*N'*-(3,5-dimethylbenzylidene)-3-hydroxy-6-(4-methoxyphenyl)pyrazine-2-carbohydrazide (Compound 15);
- (*E*)-6-(4-ethoxyphenyl)-*N'*-((2-fluoro-5-methoxypyridine-3-yl)methylene)-5-hydroxypyrazine-2-carbohydrazide (Compound 16);
- (*E*)-6-(4-ethoxyphenyl)-*N'*-((2-fluoro-5-methoxypyridine-3-yl)methylene)-3-hydroxypyrazine-2-carbohydrazide (Compound 17);
- (*E*)-6-(4-ethoxy-2-fluorophenyl)-5-hydroxy-*N'*-(3-methoxybenzylidene)pyrazine-2-carbohydrazide (Compound 18);
- (*E*)-6-(4-ethoxy-2-fluorophenyl)-3-hydroxy-*N*'-(3-methoxybenzylidene)pyrazine-2-carbohydrazide (Compound 19);
- (*E*)-*N*'-(3,5-dihydroxybenzylidene)-6-(4-methoxyphenyl)pyrazine-2-carbohydrazide (Compound 20);
- (*E*)-*N*'-(3-hydroxy-5-methoxybenzylidene)-6-(4-methoxyphenyl)pyrazine-2-carbohydrazide (Compound 21);
- (*E*)-*N'*-(4-hydroxybenzylidene)-6-(4-methoxyphenyl)pyrazine-2-carbohydrazide (Compound 22);
- (*E*)-*N'*-(3-hydroxybenzylidene)-6-(4-methoxyphenyl)pyrazine-2-carbohydrazide (Compound 23);
- (*E*)-*N'*-(2-hydroxybenzylidene)-6-(4-methoxyphenyl)pyrazine-2-carbohydrazide (Compound 24);
- (*E*)-*N'*-(3-hydroxy-5-methylbenzylidene)-6-(4-methoxyphenyl)pyrazine-2-carbohydrazide (Compound 25);
- (*E*)-6-(4-ethoxyphenyl)-*N'*-(3-hydroxy-5-methylbenzylidene)pyrazine-2-carbohydrazide (Compound 26);
- (*E*)-6-(4-ethoxyphenyl)-*N'*-(2-fluoro-5-hydroxybenzylidene)pyrazine-2-carbohydrazide (Compound 27);
- (*E*)-6-(4-ethoxyphenyl)-*N'*-(2-hydroxy-5-methoxybenzylidene)pyrazine-2-carbohydrazide (Compound 28);
- (*E*)-6-(4-ethoxyphenyl)-*N*'-((5-hydroxypyridin-3-yl)methylene)pyrazine-2-carbohydrazide (Compound 29);
- (*E*)-6-(4-ethoxyphenyl)-*N*'-((6-hydroxypyridin-2-yl)methylene)pyrazine-2-carbohydrazide (Compound 30);
- (*E*)-6-(4-ethoxyphenyl)-*N*'-(2-hydroxy-3,5-dimethoxybenzylidene)pyrazine-2-carbohydrazide (Compound 31);
- (*E*)-6-(4-ethoxyphenyl)-*N'*-((2-fluoro-5-hydroxypyridine-3-yl)methylene)pyrazine-2-carbohydrazide (Compound 32);
- (*E*)-6-(4-ethoxyphenyl)-*N'*-((2-fluoro-4-hydroxy-5-methoxypyridine-3-yl)methylene)pyrazine-2-carbohydrazide (Compound 33);
- (*E*)-6-(4-ethoxyphenyl)-*N'*-((6-hydroxy-4-methoxypyridine-2-yl)methylene)pyrazine-2-carbohydrazide (Compound 34);
- (*E*)-6-(4-ethoxyphenyl)-*N'*-((2-hydroxy-5-methoxypyridine-3-yl)methylene)pyrazine-2-carbohydrazide (Compound 35); and
- (*E*)-6-(4-ethoxyphenyl)-*N'*-((2-hydroxy-6-methoxypyridine-4-yl)methylene)pyrazine-2-carbohydrazide (Compound 36).

**3.** COMPOUND, according to any of claims 1 or 2, **characterized by** blocking voltage-gated sodium channels Nav 1.7 and/or Nav 1.8.

**4.** PHARMACEUTICAL COMPOSITION, **characterized by** comprising a therapeutically effective amount of one or more compounds of Formula (I) or of a pharmaceutically acceptable salt, hydrate, solvate, and isomer thereof, as defined in any of claims 1 to 3, and one or more pharmaceutically acceptable excipients.

5. PHARMACEUTICAL COMPOSITION, according to claim 4, **characterized by** being formulated as an oral, sublingual, nasal, parenteral, injectable, submuscular, topical, transdermal, ocular, or rectal composition.

6. USE OF FORMULA (I) COMPOUND(S), as defined in any of claims 1 to 3, **characterized by** being for preparing a drug to treat pathologies related to neuropathic pain.

7. USE, according to claim 6, **characterized by** said pathologies being selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

8. METHOD OF TREATMENT, PREVENTION, RELIEF, SUPPRESSION AND/OR CONTROL of pathologies related to neuropathic pain **characterized by the** administration of an effective amount of at least one compound of Formula (I) or a pharmaceutically acceptable salt, hydrate, solvate and isomer thereof, as defined in any of claims 1 to 3.

9. METHOD, according to claim 8, **characterized by** being for the treatment or prophylaxis of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

10. METHOD, according to any of claims 8 or 9, **characterized by** the administration of at least one compound of Formula (I) being selected from the group comprising oral, sublingual, nasal, parenteral, injectable, submuscular, topical, transdermal, ocular, and rectal routes.

11. PROCESS OF OBTAINING GENERAL FORMULA (I) COMPOUND, as defined in any of the claims 1 to 3, **characterized by** comprising the steps:

(a) formation of the Formula III intermediate:

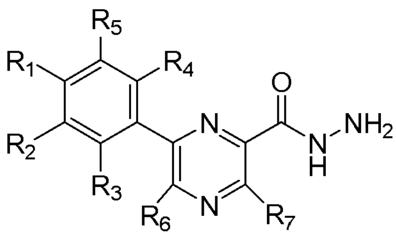

Formula III

from the hydrazinolysis reaction of a Formula IV intermediate:

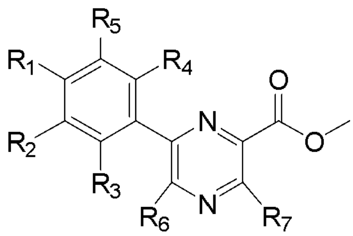

Formula IV

(b) obtaining Formula I compound;

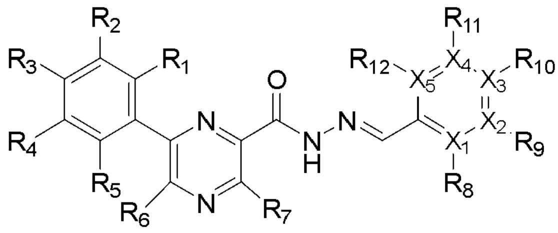

Formula (I)

from the condensation of Formula II intermediates:

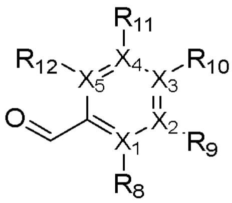

Formula II

and Formula III, with or without the presence of a catalyst and a suitable solvent;
wherein,

- one of $X_1$, $X_2$, $X_3$, $X_4$ and $X_3$ is nitrogen and the others are carbon, wherein when one of $X_1$, $X_2$, $X_3$, $X_4$ and $X_5$ is nitrogen, the corresponding R ($R_5$, $R_6$ and/or $R_7$) will be null;
- $R_1$, $R_2$, $R_3$, $R_4$, and $R_5$ are independently selected from the group consisting of hydrogen, halogen, hydroxy, alkoxy $C_1$-$C_6$ linear or branched, alkyl $C_1$-$C_5$ linear or branched, wherein at least one of $R_1$-$R_3$ is hydroxy, when $R_6$-$R_{12}$ are different from hydroxy;
- $R_6$ and $R_7$ are independently selected from the group consisting of hydrogen, halogen, hydroxy, linear or branched, $C_1$-$C_6$ alkyl, $C_1$-$C_6$ linear or branched alkoxy, wherein at least one of $R_6$-$R_7$ is hydroxy, when $R_1$-$R_3$ and $R_8$-$R_{12}$ are different from hydroxy; and
- $R_8$, $R_9$, $R_{10}$, $R_{11}$, and $R_{12}$ are independently selected from the group consisting of hydrogen, linear or branched $C_1$-$C_6$ alkoxy, alkyl linear or branched $C_1$-$C_6$, halogen, hydroxy, where at least one of $R_8$-$R_{12}$ is hydroxy, while $R_1$-$R_7$ are different from hydroxy.

**12.** PROCESS, according to claim 11, **characterized by** said catalyst of step (b) being selected from concentrated hydrochloric acid, acetic acid, trifluoroacetic acid, formic acid, or combinations thereof and said solvent being selected from dimethylformamide, dimethylsulfoxide, alcohols, or combinations thereof.

**13.** KIT, **characterized by** comprising a pharmaceutical composition, as defined in claim 4, and an application device.

## INTERNATIONAL SEARCH REPORT

International application No. **PCT/BR2024/050026**

**A. CLASSIFICATION OF SUBJECT MATTER**

***C07C 233/64***(2006.01)i; ***C07C 243/38***(2006.01)i; ***C07C 243/16***(2006.01)i; **C07C 15/00**(2006.01)i; ***C07C 15/12***(2006.01)i; **C07D 213/00**(2006.01)i; **C07D 401/00**(2006.01)i; **C07D 403/00**(2006.01)i; **A61K 31/166**(2006.01)i; ***A61P 25/02***(2006.01)i; ***A61P 25/04***(2006.01)i; **A61P 29/00**(2006.01)i

CPC: C07C 233/64, C07C 243/38, C07C 243/16, C07C 15/00, C07C 15/12; C07D 213/00, C07D 401/00, C07D 403/00, A61K 31/166, A61P 25/02; A61P 25/04, A61P 29/00

According to International Patent Classification (IPC) or to both national classification and IPC

**B. FIELDS SEARCHED**

Minimum documentation searched (classification system followed by classification symbols)

C07C 233/64; C07C 243/38; C07C 243/16; C07C 15/00; C07C 15/12; C07D 213/00; C07D 401/00; C07D 403/00; A61K 31/166; A61P 25/02; A61P 25/04; A61P 29/00

CPC: C07C 233/64, C07C 243/38, C07C 243/16, C07C 15/00, C07C 15/12; C07D 213/00, C07D 401/00, C07D 403/00, A61K 31/166, A61P 25/02; A61P 25/04, A61P 29/00

Documentation searched other than minimum documentation to the extent that such documents are included in the fields searched

Database INPI-BR; Periódicos Capes

Electronic data base consulted during the international search (name of data base and, where practicable, search terms used)

Derwent Innovation; REGISTRY, CAPLUS, MARPAT (STN®); ESPACENET

**C. DOCUMENTS CONSIDERED TO BE RELEVANT**

| Category* | Citation of document, with indication, where appropriate, of the relevant passages | Relevant to claim No. |
|---|---|---|
| P,X | WO 2023010191 A1 (EUROFARMA LABORATORIOS S A [BR]) 09 February 2023 (2023-02-09) See abstract and claims. | 1-7, 11-13 |
| P,X | WO 2023097386 A1 (EUROFARMA LABORATORIOS S A [BR]) 08 June 2023 (2023-06-08) See abstract and claims. | 1-7, 11-13 |
| Y | Lopes A.B. et al. Characterization of amide bond conformers for a novel heterocyclic template of N-acylhydrazone derivatives. Molecules. 2013 Sep 25;18(10):11683-704. doi: 10.3390/molecules181011683. PMID: 24071978; PMCID: PMC6270085. See the whole document, especially the abstract and pages 11684 and 11685. | 1-7, 11-13 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 April 2024** | **29 April 2024** |

Name and mailing address of the ISA/BR

**National Institute of Industrial Property (Brazil)**
**Rua Mayrink Veiga, 9, 6º andar, CEP 20.090-910 Rio de Janeiro – RJ**
**Brazil**

Telephone No. **(55 21) 3037-3742, 3037-3984**

Authorized officer

**Rodinelli OLIVEIRA**

Telephone No. **+55 21 3037 4528 - 3037 3319**

INTERNATIONAL SEARCH REPORT

International application No. PCT/BR2024/050026

| C. DOCUMENTS CONSIDERED TO BE RELEVANT | | |
|---|---|---|
| Category* | Citation of document, with indication, where appropriate, of the relevant passages | Relevant to claim No. |
| Y | Da Silva Y.K. et al. Synthesis and pharmacological evaluation of pyrazine N-acylhydrazone derivatives designed as novel analgesic and anti-inflammatory drug candidates. Bioorg Med Chem. 2010 Jul 15;18(14):5007-15. doi: 10.1016/j.bmc.2010.06.002. Epub 2010 Jun 8. PMID: 20598893. See the whole document, especially the abstract and pages 5007-5008 and 5014. | 1-7, 11-13 |
| Y | US 2008300240 A1 (OMEROS CORP [US]) 04 December 2008 (2008-12-04) See the whole document, especially the abstract, pages 2-10, example 7 and claims 1, 2, 3 and 10. | 1-7, 11-13 |
| Y | US 2006111409 A1 (MUTO SUSUMU [JP]) 25 May 2006 (2006-05-25) See abstract; pages 30-104; compounds 15, 304, 306 and 321; claim 1. | 1-7, 11-13 |
| Y | US 2006100257 A1 (MUTO SUSUMU [JP]) 11 May 2006 (2006-05-11) See abstract; pages 28-128; compounds 15, 304, 306 and 321; claim 1. | 1-7, 11-13 |
| Y | US 2006019958 A1 (MUTO SUSUMU [JP]) 26 January 2006 (2006-01-26) See abstract; pages 29-103; compounds 15, 304, 306 and 321; claim 1. | 1-7, 11-13 |
| Y | WO 2011069039 A1 (US HEALTH [US]) 09 June 2011 (2011-06-09) See abstract; pages 3, 4, 13-20, 30; claims 1, 14, 23. | 1-7, 11-13 |
| Y | WO 2012129562 A2 (BOUCHEZ LAURE [CH]) 27 September 2012 (2012-09-27) See abstract; claim 1. | 1-7, 11-13 |
| Y | CN 103880707 A (SHANGHAI JIAOTONG UNIV SCHOOL MEDICINE) 25 June 2014 (2014-06-25) See abstract; paragraph 0024; claims. | 1-7, 11-13 |
| Y | WO 2020023802 A1 (HOPE CITY [US]) 30 January 2020 (2020-01-30) See abstract; pages 49-84, 88-98, 174-175. | 1-7, 11-13 |
| Y | EP 1514544 A1 (INST MED MOLECULAR DESIGN INC [JP]) 16 March 2005 (2005-03-16) See abstract; pages 41-108; compounds 15, 304, 306 and 321; claim 1. | 1-7, 11-13 |
| A | Rodrigues, F.A.R. et al. Biological evaluation of pyrazinamide derivatives as an anticancer class. ECB. 2014; 3(4): 358-361. https://www.eurchembull.com/uploads/paper/c5e03c07c95123429c53bc95785161d9.pdf | 1-7, 11-13 |
| A | WO 2012080729 A2 (ELECTROPHORETICS LTD [GB]) 21 June 2012 (2012-06-21) The whole document | 1-7, 11-13 |
| A | CN 111925357 A (KUNMING INST BOTANY CAS) 13 November 2020 (2020-11-13) The whole document | 1-7, 11-13 |
| A | CN 110128343 A (SICHUAN PROVINCIAL PEOPLES HOSPITAL) 16 August 2019 (2019-08-16) The whole document | 1-7, 11-13 |
| A | WO 2013123071 A1 (CLEAVE BIOSCIENCES INC [US]) 22 August 2013 (2013-08-22) The whole document | 1-7, 11-13 |
| A | CN 105481765 A (JIANGSU AIFAN BIOLOGICAL PHARMACEUTICAL CO LTD) 13 April 2016 (2016-04-13) The whole document | 1-7, 11-13 |
| A | US 2010035932 A1 (SCHEPETKIN IGOR A [US]) 11 February 2010 (2010-02-11) The whole document | 1-7, 11-13 |
| A | WO 2004105488 A1 (BASF AG [DE]) 09 December 2004 (2004-12-09) The whole document | 1-7, 11-13 |

Form PCT/ISA/210 (second sheet) (July 2022)

**INTERNATIONAL SEARCH REPORT**

International application No.
**PCT/BR2024/050026**

**C. DOCUMENTS CONSIDERED TO BE RELEVANT**

| Category* | Citation of document, with indication, where appropriate, of the relevant passages | Relevant to claim No. |
|---|---|---|
| A | Shutske, G.M. Synthesis of Anhydro-3-mercapto-5-pyrazinyl-1,2,4-triazolium Hydroxides. J. Heterocyclic Chern., 18, 1017 (1981). https://onlinelibrary.wiley.com/doi/pdf/10.1002/jhet.5570180533 | 1-7, 11-13 |
| A | Gomes LR, Low JN, Rodrigues AS, Wardell JL, Lima CH, de Souza MV. Five N'-benzylidene-N-methylpyrazine-2-carbohydrazides. Acta Crystallogr C. 2013 May;69(Pt 5):549-55. doi: 10.1107/S0108270113010056. Epub 2013 Apr 17. PMID: 23629912. | 1-7, 11-13 |
| A | Howie, R. Alan, da Silva Lima, Camilo H., Kaiser, Carlos R., de Souza, Marcus V. N., Wardell, James L. and Wardell, Solange M. S. V. "Structures of (pyrazinecarbonyl)hydrazones of substituted benzaldehydes: different supramolecular arrangements from C—H···X (X = O, N, π) hydrogen bonds" Zeitschrift für Kristallographie, vol. 225, no. 4, 2010, pp. 158-166. https://doi.org/10.1524/zkri.2010.1235 | 1-7, 11-13 |
| A | CN 1752085 A (INST OF TOXIC PHARMACOLOGY ACA [CN]) 29 March 2006 (2006-03-29)<br>The whole document | 1-7, 11-13 |
| A | WO 03093297 A2 (EXELIXIS INC [US]) 13 November 2003 (2003-11-13)<br>The whole document | 1-7, 11-13 |
| A | WO 2010071837 A1 (VERTEX PHARMA [US]) 24 June 2010 (2010-06-24)<br>The whole document | 1-7, 11-13 |
| A | WO 2020047668 A1 (UNIV BRITISH COLUMBIA [CA]) 12 March 2020 (2020-03-12)<br>The whole document | 1-7, 11-13 |
| A | WO 2020210428 A1 (BAYLOR COLLEGE MEDICINE [US]) 15 October 2020 (2020-10-15)<br>The whole document | 1-7, 11-13 |
| A | WO 2020209932 A1 (UNIV PUERTO RICO [US]) 15 October 2020 (2020-10-15)<br>The whole document | 1-7, 11-13 |
| A | US 2015087673 A1 (HITOSHI YASUMICHI [US]) 26 March 2015 (2015-03-26)<br>The whole document | 1-7, 11-13 |
| A | WO 03051851 A1 (ASTRAZENECA AB [SE]) 26 June 2003 (2003-06-26)<br>The whole document | 1-7, 11-13 |
| A | WO 03051366 A2 (Abbott Laboratories) 26 June 2003 (2003-06-26)<br>The whole document | 1-7, 11-13 |
| A | US 2003199511 A1 (LI QUN,) 23 October 2003 (2003-10-23)<br>The whole document | 1-7, 11-13 |

Form PCT/ISA/210 (second sheet) (July 2022)

**INTERNATIONAL SEARCH REPORT**

International application No.
**PCT/BR2024/050026**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **8-10**
because they relate to subject matter not required to be searched by this Authority, namely:
Claims 8-10 contain subject matter that falls under the provisions of PCT Rule 39.1(iv) concerning methods for treatment of the human or animal body by surgery or therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/BR2024/050026**

| Patent document cited in search report | Publication date (day/month/year) | Patent family member(s) | Publication date (day/month/year) |
|---|---|---|---|
| WO 2023010191 A1 | 09 February 2023 | AR 126669 A1 | 01 November 2023 |
| | | CA 3227730 A1 | 09 February 2023 |
| | | IL 310568 A | 01 March 2024 |
| WO 2023097386 A1 | 08 June 2023 | AR 127840 A1 | 06 March 2024 |
| US 2008300240 A1 | 04 December 2008 | US 7786139 B2 | 31 August 2010 |
| | | US 2011021509 A1 | 27 January 2011 |
| | | US 8278327 B2 | 02 October 2012 |
| US 2006111409 A1 | 25 May 2006 | AU 2003242137 A1 | 22 December 2003 |
| | | CA 2488342 A1 | 18 December 2003 |
| | | CN 1658850 A | 24 August 2005 |
| | | CN 100379410 C | 09 April 2008 |
| | | CN 101103977 A | 16 January 2008 |
| | | EA 200401609 A1 | 30 June 2005 |
| | | EA 008769 B1 | 31 August 2007 |
| | | EP 1510207 A1 | 02 March 2005 |
| | | EP 1510207 A4 | 31 December 2008 |
| | | JP WO2003103648 A1 | 06 October 2005 |
| | | TW 200407107 A | 16 May 2004 |
| | | TW I280876 B | 11 May 2007 |
| | | WO 03103648 A1 | 18 December 2003 |
| US 2006100257 A1 | 11 May 2006 | AU 2003242127 A1 | 22 December 2003 |
| | | CA 2487891 A1 | 18 December 2003 |
| | | CN 1658849 A | 24 August 2005 |
| | | EA 200401607 A1 | 25 August 2005 |
| | | EA 009523 B1 | 28 February 2008 |
| | | EP 1512396 A1 | 09 March 2005 |
| | | EP 1512396 A4 | 31 December 2008 |
| | | JP WO2003103647 A1 | 06 October 2005 |
| | | TW 200410671 A | 01 July 2004 |
| | | WO 03103647 A1 | 18 December 2003 |
| US 2006019958 A1 | 26 January 2006 | AU 2003242131 A1 | 22 December 2003 |
| | | CA 2487900 A1 | 18 December 2003 |
| | | CN 1658854 A | 24 August 2005 |
| | | EA 200401608 A1 | 25 August 2005 |
| | | EA 008622 B1 | 29 June 2007 |
| | | EP 1510210 A1 | 02 March 2005 |
| | | EP 1510210 A4 | 07 January 2009 |
| | | JP WO2003103658 A1 | 06 October 2005 |
| | | TW 200407112 A | 16 May 2004 |
| | | WO 03103658 A1 | 18 December 2003 |
| WO 2011069039 A1 | 09 June 2011 | US 2012316198 A1 | 13 December 2012 |
| | | US 8518968 B2 | 27 August 2013 |
| WO 2012129562 A2 | 27 September 2012 | NONE | |
| CN 103880707 A | 25 June 2014 | CN 103880707 B | 13 April 2016 |
| WO 2020023802 A1 | 30 January 2020 | US 2021355103 A1 | 18 November 2021 |
| EP 1514544 A1 | 16 March 2005 | NONE | |
| WO 2012080729 A2 | 21 June 2012 | WO 2012080729 A3 | 02 August 2012 |
| | | WO 2012080729 A4 | 08 November 2012 |
| | | AU 2011343039 A1 | 27 June 2013 |

Form PCT/ISA/210 (patent family annex) (July 2022)

**INTERNATIONAL SEARCH REPORT**
**Information on patent family members**

International application No.
**PCT/BR2024/050026**

| Patent document cited in search report | Publication date (day/month/year) | Patent family member(s) | Publication date (day/month/year) |
|---|---|---|---|
| | | AU 2011343039 B2 | 02 March 2017 |
| | | AU 2017200812 A1 | 02 March 2017 |
| | | AU 2017200812 B2 | 03 January 2019 |
| | | CA 2818903 A1 | 21 June 2012 |
| | | CA 2818903 C | 23 March 2021 |
| | | CN 103298460 A | 11 September 2013 |
| | | CN 103298460 B | 01 June 2016 |
| | | CN 104906103 A | 16 September 2015 |
| | | CN 104906103 B | 18 May 2018 |
| | | CN 105920010 A | 07 September 2016 |
| | | DK 2835131 T3 | 04 December 2017 |
| | | EP 2651404 A2 | 23 October 2013 |
| | | EP 2651404 B1 | 14 October 2015 |
| | | EP 2651405 A2 | 23 October 2013 |
| | | EP 2835131 A1 | 11 February 2015 |
| | | EP 2835131 B1 | 06 September 2017 |
| | | ES 2553610 T3 | 10 December 2015 |
| | | ES 2650744 T3 | 22 January 2018 |
| | | HK 1190622 A1 | 11 July 2014 |
| | | HK 1214527 A1 | 29 July 2016 |
| | | JP 2014503527 A | 13 February 2014 |
| | | JP 5937102 B2 | 22 June 2016 |
| | | JP 2014503528 A | 13 February 2014 |
| | | JP 2016172757 A | 29 September 2016 |
| | | JP 6243472 B2 | 06 December 2017 |
| | | JP 2017025080 A | 02 February 2017 |
| | | US 2014018540 A1 | 16 January 2014 |
| | | US 2014031547 A1 | 30 January 2014 |
| | | US 2015209368 A1 | 30 July 2015 |
| | | US 9789111 B2 | 17 October 2017 |
| | | US 2016058745 A1 | 03 March 2016 |
| | | US 9763947 B2 | 19 September 2017 |
| | | US 2016354375 A1 | 08 December 2016 |
| | | WO 2012080727 A2 | 21 June 2012 |
| | | WO 2012080727 A3 | 23 August 2012 |
| | | WO 2012080727 A4 | 26 October 2012 |
| CN 111925357 A | 13 November 2020 | CN 111925357 B | 06 June 2023 |
| CN 110128343 A | 16 August 2019 | NONE | |
| WO 2013123071 A1 | 22 August 2013 | NONE | |
| CN 105481765 A | 13 April 2016 | NONE | |
| US 2010035932 A1 | 11 February 2010 | NONE | |
| WO 2004105488 A1 | 09 December 2004 | AR 045688 A1 | 09 November 2005 |
| | | AU 2004243492 A1 | 09 December 2004 |
| | | BR PI0410626 A | 20 June 2006 |
| | | CL 2004001296 A1 | 08 April 2005 |
| | | CN 1842272 A | 04 October 2006 |
| | | EP 1631143 A1 | 08 March 2006 |
| | | JP 2006528955 A | 28 December 2006 |
| | | KR 20060019558 A | 03 March 2006 |

Form PCT/ISA/210 (patent family annex) (July 2022)

**INTERNATIONAL SEARCH REPORT**
**Information on patent family members**

International application No.
**PCT/BR2024/050026**

| Patent document cited in search report | Publication date (day/month/year) | Patent family member(s) | Publication date (day/month/year) |
|---|---|---|---|
| | | MX PA05012226 A | 10 February 2006 |
| | | ZA 200510406 B | 31 December 2008 |
| CN 1752085 A | 29 March 2006 | CN 100355748 C | 19 December 2007 |
| | | WO 2006032173 A1 | 30 March 2006 |
| WO 03093297 A2 | 13 November 2003 | WO 03093297 A3 | 01 July 2004 |
| | | AU 2003234464 A1 | 17 November 2003 |
| | | AU 2003234464 B2 | 04 June 2009 |
| | | CA 2484209 A1 | 13 November 2003 |
| | | CA 2484209 C | 11 June 2013 |
| | | EP 1501514 A2 | 02 February 2005 |
| | | EP 1501514 A4 | 26 August 2009 |
| | | EP 1501514 B1 | 19 December 2012 |
| | | JP 2005530760 A | 13 October 2005 |
| | | JP 4901102 B2 | 21 March 2012 |
| WO 2010071837 A1 | 24 June 2010 | AR 074822 A1 | 16 February 2011 |
| | | AU 2009327357 A1 | 28 July 2011 |
| | | AU 2009327357 B2 | 16 June 2016 |
| | | AU 2009327357 C1 | 02 February 2017 |
| | | AU 2016222396 A1 | 13 October 2016 |
| | | AU 2016222396 B2 | 05 April 2018 |
| | | AU 2018201363 A1 | 15 March 2018 |
| | | AU 2018201363 B2 | 20 February 2020 |
| | | AU 2018201363 C1 | 18 June 2020 |
| | | BR PI0924084 A2 | 10 November 2020 |
| | | CA 2747252 A1 | 24 June 2010 |
| | | CA 2747252 C | 18 September 2018 |
| | | CA 3013000 A1 | 24 June 2010 |
| | | CA 3013000 C | 13 December 2022 |
| | | CN 102300862 A | 28 December 2011 |
| | | CN 102300862 B | 23 November 2016 |
| | | CN 106496209 A | 15 March 2017 |
| | | CN 106496209 B | 30 June 2020 |
| | | CN 106496210 A | 15 March 2017 |
| | | CN 106496210 B | 09 June 2020 |
| | | CN 106518856 A | 22 March 2017 |
| | | CN 106518856 B | 28 April 2020 |
| | | CY 1120002 T1 | 12 December 2018 |
| | | DK 2376485 T3 | 12 March 2018 |
| | | DK 3354650 T3 | 30 May 2022 |
| | | EP 2376485 A1 | 19 October 2011 |
| | | EP 2376485 B1 | 06 December 2017 |
| | | EP 3354650 A1 | 01 August 2018 |
| | | EP 3354650 B1 | 16 February 2022 |
| | | EP 4059932 A1 | 21 September 2022 |
| | | ES 2663222 T3 | 11 April 2018 |
| | | ES 2921576 T3 | 29 August 2022 |
| | | HK 1259148 A1 | 29 November 2019 |
| | | HR P20180385 T1 | 06 April 2018 |
| | | HR P20220581 T1 | 10 June 2022 |
| | | HU E036068 T2 | 28 June 2018 |

Form PCT/ISA/210 (patent family annex) (July 2022)

**INTERNATIONAL SEARCH REPORT**
**Information on patent family members**

International application No.
**PCT/BR2024/050026**

| Patent document cited in search report | Publication date (day/month/year) | Patent family member(s) | | | Publication date (day/month/year) |
|---|---|---|---|---|---|
| | | IL | 213613 | A | 29 January 2015 |
| | | IL | 236602 | B | 31 July 2018 |
| | | IL | 260158 | A | 31 July 2018 |
| | | IL | 260158 | B | 29 October 2020 |
| | | JP | 2012512906 | A | 07 June 2012 |
| | | JP | 5753093 | B2 | 22 July 2015 |
| | | JP | 2015024996 | A | 05 February 2015 |
| | | JP | 5894643 | B2 | 30 March 2016 |
| | | JP | 2016030756 | A | 07 March 2016 |
| | | JP | 6212527 | B2 | 11 October 2017 |
| | | JP | 2018009010 | A | 18 January 2018 |
| | | JP | 6577537 | B2 | 18 September 2019 |
| | | JP | 2019194250 | A | 07 November 2019 |
| | | KR | 20110096158 | A | 29 August 2011 |
| | | KR | 101745331 | B1 | 09 June 2017 |
| | | KR | 20150003924 | A | 09 January 2015 |
| | | KR | 101755216 | B1 | 07 July 2017 |
| | | KR | 20170081739 | A | 12 July 2017 |
| | | KR | 101958632 | B1 | 15 March 2019 |
| | | LT | 2376485 | T | 26 March 2018 |
| | | LT | 3354650 | T | 27 June 2022 |
| | | MX | 2011006503 | A | 06 September 2011 |
| | | NO | 2376485 | T3 | 05 May 2018 |
| | | NZ | 593969 | A | 29 November 2013 |
| | | PL | 2376485 | T3 | 30 May 2018 |
| | | PL | 3354650 | T3 | 13 June 2022 |
| | | PT | 2376485 | T | 12 March 2018 |
| | | PT | 3354650 | T | 20 June 2022 |
| | | RS | 56995 | B1 | 31 May 2018 |
| | | RU | 2011129761 | A | 20 May 2013 |
| | | RU | 2604066 | C2 | 10 December 2016 |
| | | RU | 2016141566 | A | 20 December 2018 |
| | | RU | 2016141566 | A3 | 17 February 2020 |
| | | SG | 10201607592P | A | 29 November 2016 |
| | | SG | 172248 | A1 | 28 July 2011 |
| | | SI | 2376485 | T1 | 30 April 2018 |
| | | SI | 3354650 | T1 | 30 June 2022 |
| | | TW | 201028404 | A | 01 August 2010 |
| | | TW | I478918 | B | 01 April 2015 |
| | | TW | 201520210 | A | 01 June 2015 |
| | | TW | I543979 | B | 01 August 2016 |
| | | TW | 201708213 | A | 01 March 2017 |
| | | TW | I643854 | B | 11 December 2018 |
| | | US | 2019047995 | A1 | 14 February 2019 |
| | | US | 10479784 | B2 | 19 November 2019 |
| | | US | 2018170922 | A1 | 21 June 2018 |
| | | US | 10961232 | B2 | 30 March 2021 |
| | | US | 2010222318 | A1 | 02 September 2010 |
| | | US | 8841308 | B2 | 23 September 2014 |
| | | US | 2015031661 | A1 | 29 January 2015 |

Form PCT/ISA/210 (patent family annex) (July 2022)

**INTERNATIONAL SEARCH REPORT**
**Information on patent family members**

International application No.
**PCT/BR2024/050026**

| Patent document cited in search report | Publication date (day/month/year) | Patent family member(s) | Publication date (day/month/year) |
|---|---|---|---|
| | | US 9365557 B2 | 14 June 2016 |
| | | US 2015051187 A1 | 19 February 2015 |
| | | US 9701674 B2 | 11 July 2017 |
| | | US 2016311809 A1 | 27 October 2016 |
| | | US 2023030414 A1 | 02 February 2023 |
| | | ZA 201104498 B | 31 October 2012 |
| WO 2020047668 A1 | 12 March 2020 | US 2022112157 A1 | 14 April 2022 |
| | | US 2023134821 A1 | 04 May 2023 |
| WO 2020210428 A1 | 15 October 2020 | NONE | |
| WO 2020209932 A1 | 15 October 2020 | US 2023167071 A1 | 01 June 2023 |
| US 2015087673 A1 | 26 March 2015 | WO 2015048547 A2 | 02 April 2015 |
| | | WO 2015048547 A3 | 18 June 2015 |
| WO 03051851 A1 | 26 June 2003 | AR 038044 A1 | 22 December 2004 |
| | | AT E354570 T1 | 15 March 2007 |
| | | AU 2002352425 A1 | 30 June 2003 |
| | | BR 0214989 A | 14 December 2004 |
| | | CA 2469786 A1 | 26 June 2003 |
| | | CN 1620438 A | 25 May 2005 |
| | | CO 5590917 A2 | 30 December 2005 |
| | | DE 60218340 D1 | 05 April 2007 |
| | | DE 60218340 T2 | 29 November 2007 |
| | | EP 1458690 A1 | 22 September 2004 |
| | | EP 1458690 B1 | 21 February 2007 |
| | | ES 2280599 T3 | 16 September 2007 |
| | | HU P0402026 A2 | 28 February 2005 |
| | | HU P0402026 A3 | 29 August 2005 |
| | | IS 7314 A | 16 June 2004 |
| | | JP 2005517655 A | 16 June 2005 |
| | | KR 20040068286 A | 30 July 2004 |
| | | MX PA04005990 A | 27 September 2004 |
| | | NO 20043022 L | 15 July 2004 |
| | | NZ 533275 A | 24 February 2006 |
| | | PL 369731 A1 | 02 May 2005 |
| | | RU 2004116916 A | 10 November 2005 |
| | | SE 0104330 D0 | 19 December 2001 |
| | | TW 200410694 A | 01 July 2004 |
| | | US 2005032808 A1 | 10 February 2005 |
| | | US 7342019 B2 | 11 March 2008 |
| | | ZA 200404805 B | 15 August 2005 |
| WO 03051366 A2 | 26 June 2003 | NONE | |
| US 2003199511 A1 | 23 October 2003 | US 6831175 B2 | 14 December 2004 |
| | | US 2003187026 A1 | 02 October 2003 |
| | | WO 03051366 A2 | 26 June 2003 |
| | | WO 03051366 A3 | 25 March 2004 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10550080 B
- US 9765029 B
- US 10000475 B
- WO 2020261114 A
- WO 2020092667 A
- US 9163042 B
- WO 2014120808 A
- WO 2014120815 A
- WO 2018213426 A
- WO 2019014352 A
- WO 2015006280 A
- US 7928107 B
- WO 2018235851 A
- US 8629149 B
- JP 2017001991 B


### Non-patent literature cited in the description


- **SMITH**. *Pain*, 2020, vol. 161 (1), S127
- **CAVALLI**. *Int. J. Immunopathol. Pharmacol*, 2019, vol. 33, 2058738419838383
- **BOUHASSIRA**. *Rev Neurol*, 2019, vol. 175 (1-2), 16
- **SCHOLZ**. *Nature Neurosci.*, 2002, vol. 5 (10), 62
- **COSTIGAN**. *Annu. Rev. Neurosci.*, 2009, vol. 32, 1
- **DWORKIN**. *Clin. J. Pain*, 2002, vol. 18 (6), 343
- **DUCREUX**. *Brain*, 2006, vol. 129, 963
- **PAK**. *Curr. Pain Headache Rep*, 2018, vol. 22 (2), 9
- **KUSHNAREV**. *Expert Opin. Investig. Drugs*, 2020, vol. 29 (3), 259
- **EMERY**. *Expert Opin. Ther. Targets*, 2016, vol. 20 (8), 975
- **CATTERALL**. *Nat. Chem. Biol.*, 2020, vol. 16, 1314
- **WISEDCHAISRI**. *Cell*, 2019, vol. 178 (4), 993
- **CLAIRFEUILLE**. *Science*, 2019, vol. 363, 1302
- **LERA-RUIZ**. *J. Med. Chem.*, 2015, vol. 58 (18), 7093
- **BAGAL**. *J. Med. Chem.*, 2013, vol. 56 (3), 593
- **BAGAL**. *Channels*, 2015, vol. 9 (6), 360
- **LAW**. *Drug Discovery Today*, 2019, vol. 24 (7), 1389
- **BAGAL**. *Channels (Austin)*, 2015, vol. 9 (6), 360
- **VETTER**. *Pharmacology & Therapeutics*, 2017, vol. 172, 73
- **AHUJA**. *Science*, 2015, vol. 350 (6267), 1491
- **KINGWELL**. *Nat. Rev. Drug Discov.*, 2019, vol. 18, 321
- **SAFINA**. *J. Med. Chem.*, 2021, vol. 64, 2953
- **LUO**. *J. Med. Chem.*, 2019, vol. 62, 831
- **BANKAR**. *Cell Reports*, 2018, vol. 24, 3133
- **BROWN**. *Bioorg. Med. Chem.*, 2019, vol. 2 (1), 230
- **PAYNE**. *Br. J. Pharmacol.*, 2015, vol. 172 (10), 2654
- **BAGAL**. *Med. Chem. Lett*, 2015, vol. 6 (6), 650
- **KORT**. *J. Med. Chem.*, 2008, vol. 51, 407
- **ZHANG**. *Neuropharmacology*, 2010, vol. 59, 201-207
- **KORNECOOK**. *J. Pharmacol. Exp. Ther.*, 2017, vol. 362, 146
- **DEUIS**. *Neuropharmacology*, 2017, vol. 127, 87-108
- **MCKERRALL**. *Bioorganic & Medicinal Chemistry Lett.*, 2018, vol. 28, 3141
- **BAGAL**. *Bioorganic & Medicinal Chemistry Lett.*, 2014, vol. 24, 3690